(19) **Europäisches Patentamt**

**European Patent Office**

**Office européen des brevets**

(11) **EP 1 724 583 A1**

(12) **EUROPEAN PATENT APPLICATION**
published in accordance with Art. 158(3) EPC

(43) Date of publication:
**22.11.2006 Bulletin 2006/47**

(51) Int Cl.:
***G01N 33/543*** (2006.01)

(21) Application number: **05720505.6**

(22) Date of filing: **10.03.2005**

(86) International application number:
**PCT/JP2005/004234**

(87) International publication number:
**WO 2005/088309 (22.09.2005 Gazette 2005/38)**

(84) Designated Contracting States:
**DE ES FR GB IT**

(30) Priority: **12.03.2004 JP 2004070828**
**23.04.2004 JP 2004128050**

(71) Applicant: **Pulse-Immunotech Corporation**
**Hachioji-shi, Tokyo 192-0982 (JP)**

(72) Inventors:
• **IWATA, Keisuke,**
**Tokyo University of Technology**
**Hachioji-shi,**
**Tokyo 192-0982 (JP)**

• **MIZUTANI, Yukihito,**
**Tokyo University of Technology**
**Hachioji-shi,**
**Tokyo 192-0982 (JP)**

(74) Representative: **Grünecker, Kinkeldey,**
**Stockmair & Schwanhäusser**
**Anwaltssozietät**
**Maximilianstrasse 58**
**80538 München (DE)**

(54) **METHOD OF MEASURING AFFINITY SUBSTANCE**

(57) The binding reaction between an affinity substance to be measured and a binding partner having the activity to bind to the affinity substance is measured using agglutination reaction. The binding partner is bound to carrier particles, and the carrier particles agglutinate through a binding reaction. According to the present invention, the agglutination reaction is enhanced by incubating the reaction solution before an electric field is applied. According to the present invention, the agglutination reaction is also enhanced by adjusting the temperature and/or viscosity of the reaction solution placed in an electric field. The present invention contributes to the improvement of measurement sensitivity. The present invention also comprises the step of diluting the carrier particle-containing reaction solution before counting the agglutinates. The binding between the affinity substance and the binding partner thereof is enhanced in the dilution step of the present invention. As a result, the disruption of agglutinates is prevented and the measurement sensitivity is increased. Furthermore, diluted carrier particles and agglutinates can be distinguished with high accuracy.

**Description**

Technical Field

The present invention relates to methods and devices for measuring substances having affinity (also referred to as "affinity substances") using agglutination reactions of carrier particles.

Background Art

Conventional methods for detecting or measuring the presence of affinity substances include, for example, enzyme immunoassays and radioimmunoassays. The affinity substances are measured based on the binding levels between the affinity substances and binding partners thereof in any methods. These are highly sensitive and accurate methods. However, their reagents are unstable because enzymes or radioisotopes are used as labels. Furthermore, these assays that use radioisotopes require meticulous attention to detail and technical skills because there are regulations for radioisotope storage and preservation. Thus, there has been a need for more convenient measurement methods. Furthermore, since these methods require a relatively long time for measurement, they cannot be applied for urgent tests. Under these circumstances, extensive studies on rapid and highly sensitive measurement methods were begun.

Since 1970, analysis methods that use agglutination of carrier particles as an indicator for measuring binding between affinity substances and binding partners have been put into practical use. In these methods, quantitative analysis is enabled by optical measurement of the degree of carrier particle agglutination. For example, the optical methods that use latex particles as a carrier particle for measuring immunological particle agglutination reactions are called latex agglutination turbidimetry. In general, the reaction temperature in these analysis methods ranges from 37 to 45°C, and specific agglutination reactions proceed upon mixing with a stirring impeller or such. Since the time required for measurement (reaction) ranges from about 10 to 20 minutes, these methods are more rapid than enzyme immunoassays or radioimmunoassays. However, these methods are said to be inferior to enzyme immunoassays or such in sensitivity and measurement range.

Methods for determining the particle size distribution in latex agglutination methods are also known (Non-patent Document 1, Cambiaso et al., J. Immunol. Methods 18, 33, 1977; Non-patent Document 2, Matsuzawa et al., Kagaku to kogyo (Chemistry and Chemical Industry), Vol. 36, No. 4, 1982). In latex agglutination turbidimetry, light transmittance through particle suspensions is determined by measuring the state and the number of individually dispersed particles by methods that determine particle size distribution. In the report of Cambiaso et al., an antigen was reacted with a reagent of antibody-bound latex particles (0.8 $\mu$m diameter) at 37°C for 20 minutes. The particles were counted after the reaction and the antigen was quantified based on the level of decrease in the number of particles due to agglutination. The number of particles was determined using a counter that is based on the principle of laser light scattering.

Meanwhile, Matsuzawa et al. incubated an antigen with a reagent of antibody-bound latex particles (1 $\mu$m diameter) for 6 hours. After the reaction, mean particle volume was determined by an electric resistance method to quantify the antigen. However, only the PAMIA system (SYSMEX CORPORATION), which uses a laser scattering sheath flow method, has been put into practical use and is widely used. PAMIA uses latex particles that have a diameter of 0.78 $\mu$m. Immunoassay is carried out by counting latex particles after a 15-minute reaction at 45°C. PAMIA is more sensitive than latex agglutination turbidimetry. However, PAMIA is said to be inferior in sensitivity when compared to high sensitivity immunoassay methods such as radioimmunoassays (RIA) and enzyme immunoassays (EIA).

In general, latex agglutination turbidimetry uses latex particles that have a diameter of 0.05 to 0.6 $\mu$m. When such small particles are used, methods for analyzing particle size distribution in latex agglutination are easily affected by substances that interfere with measurement. For example, lipids, proteins, blood cell components, and such coexist in body fluids such as blood and urine. These coexisting substances are indistinguishable from carrier particles, and may lead to inaccurate counting of carrier particles. Hence, relatively large particles have been used to avoid the impact of interfering substances of measurement. In contrast, agglutination reactions hardly take place when particles having a diameter of about 1 $\mu$m, such as those in Matsuzawa et al. are used. This is the reason why latex particles with a diameter of about 0.8 $\mu$m have been so far used. The diameter of the aperture (small hole) that Matsuzawa et al. used to measure mean particle volumes was 30 $\mu$m. Apertures of this size are more susceptible to clogging. However, 0.8- to 1-$\mu$m particles cannot be detected when the aperture diameter is greater than 30 $\mu$m.

In addition, a method for applying an alternating voltage to a reaction system to accelerate agglutination of carrier particles based on the binding between an affinity substance and a binding partner and to facilitate detection of the agglutinates to be formed is known (Patent Document 1, Japanese Patent Application Kokai Publication No. (JP-A) H7-83928 (unexamined, published Japanese patent application)). This method is for detecting or measuring the presence of an affinity substance based on carrier particle agglutination, and comprises applying an alternating voltage to a reaction system so that an electric field strength of 5 to 50 V/mm may be obtained in the presence of a salt of 10 mM or more.

When placed in an electric field, carrier particles which carry a binding partner align along the electric field (pearl chain formation). When the electric field is subsequently terminated, the aligned carrier particles re-disperse. When pearl chains are formed in the presence of an affinity substance, the binding partner will bind to the affinity substance. As a result, re-dispersion of the carrier particles does not take place even after termination of the electric field and the presence of pearl-chain carriers can still be observed. This phenomenon is applied to the measurement described above. That is, the affinity substance reaction is accelerated in an electric field. By allowing the carrier particles to re-disperse after the termination of electric field, agglutinates of carrier particles can be detected as the reaction product. [Patent Document 1] Japanese Patent Application Kokai Publication No. H 7-83928. [Non-patent Document 1] Cambiaso et al., J. Immunol. Methods 18, 33, 1977.

[Non-patent Document 2] Matsuzawa et al., Kagaku To Kogyo (Chemistry and Chemical Industry), Vol. 36, No. 4, 1982.


Disclosure of the Invention


Problems to be Solved by the Invention


An objective of the present invention is to provide methods of accelerating the binding between an affinity substance and a binding partner in methods that agglutinate carrier particles through the binding between an affinity substance and a binding partner. Alternatively, an objective of the present invention is to provide methods for suppressing effects of inhibitory factors on the binding of the two. In methods that measure affinity substances by applying an electric field, reaction of the affinity substances takes place when binding partner-carrying carrier particles form pearl chains. The carrier particles which agglutinate via the reaction of the two are detected or measured as an indicator of the binding. It is believed that improvement of reaction efficiency can be attained if the reaction of the two can be accelerated. Alternatively, it is useful to elucidate inhibitory factors of the reaction and to provide methods for eliminating their effects.

Another objective of the present invention is to provide methods of measuring an affinity substance that are less susceptible to dilution, for detecting agglutinates that are formed by pearl chain formation, as well as devices therefor. As described above, in methods that measure an affinity substance by counting agglutinates of carrier particles, the outcome of differentiating between agglutinates and unagglutinated carrier particles greatly affects the measurement results. Even for carrier particles that have not formed agglutinates, there is a possibility that they may be counted as agglutinates if they are in a positional relationship that makes them appear overlapped. The present inventors have observed that the issue of overlapping particles can be solved by counting agglutinated particles based on the particles' three-dimensional information. However, even in analyses that are based on such three-dimensional information, multiple particles may be counted at the same time under high concentration conditions. Namely, it has been confirmed that multiple unagglutinated carrier particles may be counted as an agglutinate.

If particles are set to a low concentration in advance, overlapping of the particles may be avoided when identifying agglutinated particles. Under conditions of low particle concentration, however, pearl chain formation is difficult. A certain level of particle concentration is necessary for particle agglutination reactions via pearl chain formation. In fact, it would be ideal if particle concentration can be increased in the process of pearl chain formation and then lowered in the process of detecting agglutinated particles. To lower particle concentration, the carrier particle-containing reaction solution may be diluted, for example.

In practice, however, diluting the reaction solution leads to disruption of formed agglutinates. As a result, the number of agglutinated particles is counted to be less than the reality. In fact, the dilution results in minus errors. The present invention provides methods of measuring an affinity substance that are less susceptible to dilution for detecting agglutinates that are formed by pearl chain formation, as well as devices therefor.


Means to Solve the Problems


The present inventors have discovered methods for improving the binding efficiency between an affinity substance and a binding partner, and thereby completed the present invention. In order to allow a binding partner carried by pearl-chain carrier particles to efficiently bind to an affinity substance, conditions under which the affinity substance can make as much contact as possible with the binding partner may be provided. In other words, conditions under which more of the affinity substance fails to make contact with a binding partner may be referred to as conditions of poor reaction efficiency. Under such conditions, improvement in the measurement sensitivity may be possibly inhibited in methods that measure the binding of the two based on carrier particle agglutination. This issue is solved by the present invention.

In addition, the temperature of a reaction solution to which a voltage is applied will increase by Joule heat. The present inventors analyzed the effects of the reaction solution temperature on the affinity substance-binding partner reaction. As a result, the present inventors observed that a rise in the reaction solution temperature may inhibit the binding of the two. The present inventors showed that the optimum reaction conditions may be achieved by regulating the temperature condition of the reaction solution to which an electric field is applied, and thereby completed the present invention. More specifically, the present invention provides the following measurement methods, measurement devices, and methods for accelerating the agglutination of binding partner-carrying carrier particles by an affinity substance.

[1] A method for measuring an affinity substance, which comprises the steps of:

(1) incubating a mixed reaction solution comprising the affinity substance to be measured and carrier particles that are bound to a binding partner having the activity to bind to the affinity substance to be measured;
(2) applying voltage pulses to the reaction solution of step (1);
(3) counting, after step (2), agglutinates of carrier particles formed through the binding with the affinity substance to be measured, or unagglutinated carrier particles that do not bind to the affinity substance to be measured, or both; and
(4) determining, after step (3), the level of the substance to be measured based on either or both of the level of agglutinate formation and the level of unagglutinated carrier particles.

[2] The method of [1], wherein the reaction solution is incubated at 37 to 90°C in step (1).
[3] The method of [2], wherein the reaction solution is incubated at 40 to 90°C in step (1).
[4] The method of [1], wherein the reaction solution contains a water-soluble polymer.
[5] The method of [1], wherein the viscosity of the reaction solution is adjusted to 0.8 to 3 mPas in step (2).
[6] The method for measuring an affinity substance according to [1], wherein step (2) is carried out at 0 to 20°C.
[7] The method for measuring an affinity substance according to [6], wherein step (2) is carried out at 0 to 10°C.
[8] The method of [1]; wherein either or both of the agglutinates and unagglutinated carrier particles are counted using the three-dimensional information thereof as an indicator.
[9] The method of [1], wherein the binding between the affinity substance and the binding partner is an antigen-antibody reaction.
[10] The method of [9], wherein the affinity substance is an antigen and the binding partner is an antibody or a fragment comprising an antigen-binding domain of the antibody.
[11] The method of [9], wherein the affinity substance is an antibody or a fragment comprising an antigen-binding domain of the antibody, and the binding partner is an antigen or a fragment comprising an epitope of the antigen.
[12] The method of [1], wherein the voltage pulse is an alternating voltage pulse.
[13] The method for measuring an affinity substance, which comprises the steps of:

(1') incubating a reaction solution comprising an affinity substance to be measured and carrier particles that are bound to a binding partner having the activity to bind to at least the affinity substance to be measured before or after mixing with an agglutination reagent, wherein the carrier particles agglutinate via the agglutination reagent and the agglutination is inhibited by the affinity substance to be measured;
(2') applying voltage pulses to the reaction solution of step (1') in the presence of the agglutination reagent;
(3') counting, after step (2'), agglutinates of carrier particles formed through the binding with the agglutination reagent, or unagglutinated carrier particles whose agglutination is inhibited by the binding of the affinity substance to be measured, or both; and
(4') determining, after step (3'), the level of the substance to be measured based on either or both of the level of agglutinate formation and the level of unagglutinated carrier particles.

[14] The method of [13], wherein, after incubation of the reaction solution in step (1'), the agglutination reagent is mixed before step (2').
[15] The method of [13], which comprises, after mixing the agglutination reagent, another incubation step before step (2').
[16] The method of [13], wherein step (2') is carried out after the reaction solution is incubated in the presence of the agglutination reagent in step (1').
[17] A device for agglutinating carrier particles, which comprises in a device a means for applying voltage pulses to a reaction solution comprising a particular substance and carrier particles that are bound to a binding partner having the activity to bind to the particular substance, a means of heating the reaction solution to a temperature within the range of 3 7 to 90°C.
[18] A method for agglutinating carrier particles, which comprises in a method of applying voltage pulses to a reaction solution comprising a particular substance and carrier particles that bind to a binding partner having the activity to bind to the particular substance, keeping the temperature of the reaction solution within the range of 0 to 20°C during voltage application.
[19] The method of [18], wherein the binding between the binding partner and the particular substance is an antigen-antibody reaction.

[20] The method of [18], wherein the voltage pulse is an alternating voltage pulse.

[21] The method of [18], wherein the water-soluble polymer is added to the reaction solution.

[22] The method of [18], wherein the viscosity of the reaction solution is adjusted to 0.8 to 3 mPas.

[23] The method of [18], which comprises the strep of incubating the carrier particles and the particular substance at 37 to 90°C before voltage pulse application.

[24] A device for agglutinating carrier particles, which comprises in a device a means for applying voltage pulses to a reaction solution comprising a particular substance and carrier particles that bind to a binding partner having the activity to bind to the particular substance, a means of keeping the temperature of the reaction solution within the range of 0 to 20°C during voltage application.

[25] A device for measuring the binding between an affinity substance and carrier particles that bind to a binding partner having the activity to bind to the affinity substance to be measured using as an indicator the agglutination of the carrier particles by the affinity substance or an agglutination reagent, comprising the elements of:

    a: a space for retaining a reaction solution;

    b: a means for incubating the reaction solution at 37 to 90°C;

    c: a means for applying voltage pulses to the reaction solution;

    d: a means for keeping the temperature of the reaction solution within the range of 0 to 20°C during voltage pulse application; and

    e: a means for counting either or both of carrier particles and agglutinates of carrier particles in the reaction solution.

The present inventors studied the step of detecting agglutinates of carrier particles extensively. The present inventors then assumed that disadvantages associated with dilution could be eliminated by the use of a means for strengthening the bonds that form agglutinates in the step of diluting the reaction solution after pearl chain formation. Further, the present inventors discovered an effective means for preventing dilution-associated disadvantages and confirmed its effectiveness, and thereby cmpleted the present invention. More specifically, the present invention relates to the following measurement methods and measurement devices.

[26] A method for diluting the reaction solution using a means of enhancing the binding between an affinity substance and a binding partner or the binding between an agglutination reagent and an binding partner before step (2) or (2') in a method of measuring an affinity substance, which comprises the steps of:

    (1) applying voltage pulses to a mixed reaction solution comprising the affinity substance to be measured and carrier particles that bind to a binding partner having the activity to bind to the affinity substance to be measured;

    (2) counting, after step (1), agglutinates of carrier particles formed through the binding with the affinity substance to be measured, or unagglutinated carrier particles that have not bound to the affinity substance to be measured, or both; and

    (3) determining, after step (2), the level of the substance to be measured based on either or both of the level of agglutinate formation and the level of unagglutinated carrier particles; or the steps of:

    (1') applying voltage pulses to a mixed reaction solution comprising an agglutination reagent component, the affinity substance to be measured, and carrier particles that bind to a binding partner having the activity to bind to the affinity substance to be measured, wherein the carrier particles agglutinate via the agglutination reagent and the agglutination is inhibited by the affinity substance to be measred;

    (2') counting, after step (1'), agglutinates of carrier particles formed by binding to the agglutination reagent, or unagglutinated carrier particles of which agglutination is inhibited by the binding of the affinity substance to be measured, or both;

    (3') determining, after step (2'), the level of the substance to be measured based on either or both of the level of agglutinate formation and the level of unagglutinated carrier particles.

[27] The method of [26], wherein the step of diluting the reaction solution mixes the reaction solution with a diluent under the condition of voltage pulse application.

[28] The method of [27], wherein the voltage pulse is an alternating voltage.

[29] The method of [28], wherein the frequency of the alternating voltage is in the range of 2 KHz to 20 MHz.

[30] The method of [27], wherein the step of diluting the reaction solution further comprises the step of mixing the reaction solution with a diluent under the condition of voltage pulse application and further diluting the carrier particles after termination of the electric field.

[31] The method of [27], wherein the step of diluting the reaction solution is a step of diluting the reaction by mixing the reaction solution after addition of a binding enhancer that enhances the binding between the affinity substance to be measured and the binding partner, or the binding between the agglutination reagent and the binding partner, or a step of diluting the reaction solution with a diluent that contains the binding enhancer.

[32] The method of [26], wherein the step of diluting the reaction solution is a step of diluting the reaction solution by mixing the reaction solution with a diluent after adding to the reaction solution a binding enhancer that enhances the binding between the affinity substance to be measured and the binding partner, or the binding between the agglutination reagent and the binding partner, or a step of diluting the reaction solution with a diluent that contains the binding enhancer.

[33] The method of [32], wherein the binding between the affinity substance to be measured and the binding partner, or the binding between the agglutination reagent and the binding partner is immunological binding.

[34] The method of [33], wherein the antigen is a protein antigen and the binding enhancer is a compound that comprises either glutaraldehyde or carbodiimide, or both.

[35] The method of [32], wherein the step of diluting the reaction solution mixes the reaction solution with a diluent during voltage pulse application.

[36] The method of [26], wherein the voltage pulse in step (1) or (1') is an alternating voltage pulse.

[37] The method of [26], wherein voltage pulses are applied several times in step (1) or (1').

[38] The method of [37], wherein step (1) or (1') comprises dispersing carrier particles and applying subsequent voltage pulses after voltage pulse application.

[39] The method of [37], wherein the voltage pulses are applies several times in different directions.

[40] The method of [26], wherein the mean particle size of carrier particles is 1 $\mu$m or greater.

[41] The method of [40], wherein the mean particle size of carrier particles is in the range of 1 to 20 $\mu$m.

[42] The method of [26], wherein step (2) or (2') counts either or both of agglutinates and unagglutinated carrier particles using three-dimensional information thereof as an indicator.

[43] The method of [42], wherein step (2) or (2') physically measures the three-dimensional information of the agglutinates or carrier particles.

[44] The method of [43], wherein the method that physically measures the three-dimensional information is any one selected from the group consisting of electric resistance method, laser diffraction method, and three dimensional imaging analysis.

[45] A device for measuring the binding between an affinity substance and carrier particles that bind to a binding partner having the activity to bind to the affinity substance to be measured, using as an indicator agglutination of the carrier particles by the affinity substance or an agglutination reagent, which comprises the elements of:

a: a space for retaining a reaction solution which comprises a sample comprising the affinity substance to be measured and carrier particles that bind to a binding partner having the activity to bind to the affinity substance to be measured, or the reaction solution further comprising an agglutination reagent;

b: a means of applying voltage pulses to the reaction solution;

c: a means of diluting the reaction solution; and

d: a means of counting either or both of carrier particles and carrier particle agglutinates in the reaction solution.

[46] The device of [45], wherein the means of diluting the reaction solution is a means of mixing the reaction solution with a diluent during voltage pulse application.

[47] The device of [45], wherein the means of diluting the reaction solution comprises a means of adding to the reaction solution a binding enhancer that enhances the binding between the affinity substance to be measured and the binding partner, or the binding between an agglutination reagent and the binding partner.

Effects of the Invention

The present invention provides methods for accelerating the binding reaction between binding partner-carrying carrier particles and affinity substances in a reaction solution to which an electric field has been applied and methods for suppressing effects of inhibitory factors on such reactions. There are not many reports to suggest optimum reaction conditions in the measurement of affinity substances using agglutination of carrier particles as an indicator. According to the present invention, for example, increase in sensitivity or reduction of reaction time may be achieved for methods of measurement that are based on immunological binding reactions using carrier particle agglutination as an indicator. The present invention contributes to the optimization of the reaction described above.

The present invention can be used to achieve, for example, increase in sensitivity or improvement in reproducibility in methods of measurement that are based on immunological binding reactions using carrier particle agglutination as an indicator. The present invention contributes to the optimization of the reaction described above. In reaction solutions to which a voltage pulse has been applied, agglutinates are formed through the binding reaction between a binding partner which is carried by carrier particles and an affinity substance (or an agglutination reagent). For detection of agglutinates in the present invention, agglutinates were detected after the reaction solution is diluted by a means of enhancing the binding reaction. As a means for enhancing the binding reaction, reaction solutions can be diluted under the condition of voltage pulse application, or by using a binding enhancer. By adopting a means for enhancing the binding reaction, various problems associated with the dilution of agglutinates may be avoided. Namely, diluted carrier particles are less likely to be detected as overlapping with each other. As a result, errors from incorrectly detecting overlapping particles as agglutinates may be prevented. Meanwhile, the conformation of agglutinates is maintained by enhancement of the binding. As such, the problem that agglutinates become disrupted and undetectable as a result of dilution can be avoided. Thus, according to the present invention, improvement in reproducibility and sensitivity can be achieved.

Brief Description of the Drawings

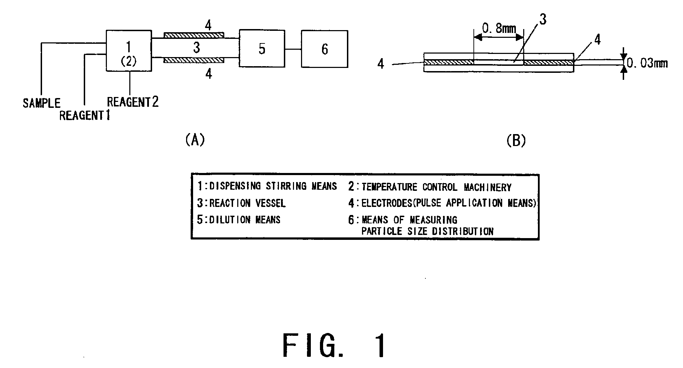

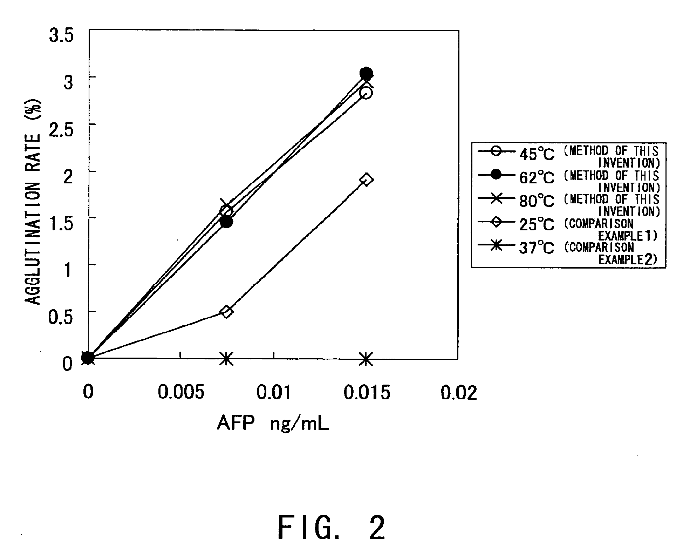

Fig. 1 (A) is a diagram illustrating the configuration of a device based on the present invention and (B) is a diagram illustrating a cross-sectional view of a pulse application vessel that composes a device based on the present invention. Fig. 2 is a diagram showing the results of measurement (in relation to pretreatment temperature) obtained by performing the measurement method of the present invention using a measurement device with the configuration of Fig. 1. In the diagram, the vertical axis and the horizontal axis represent percent agglutination (%) and AFP concentration (ng/mL) respectively.

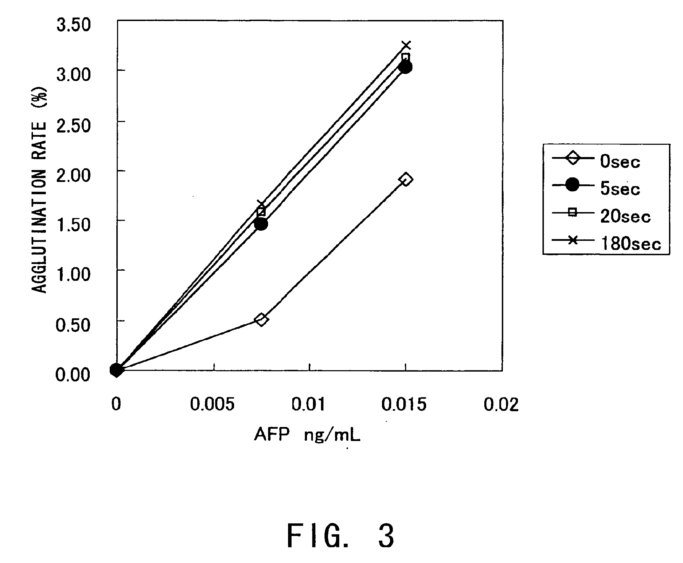

Fig. 3 is a diagram showing the results of measurement (in relation to pretreatment period at high temperature) obtained by performing the measurement method of the present invention using a measurement device with the configuration of Fig. 1.

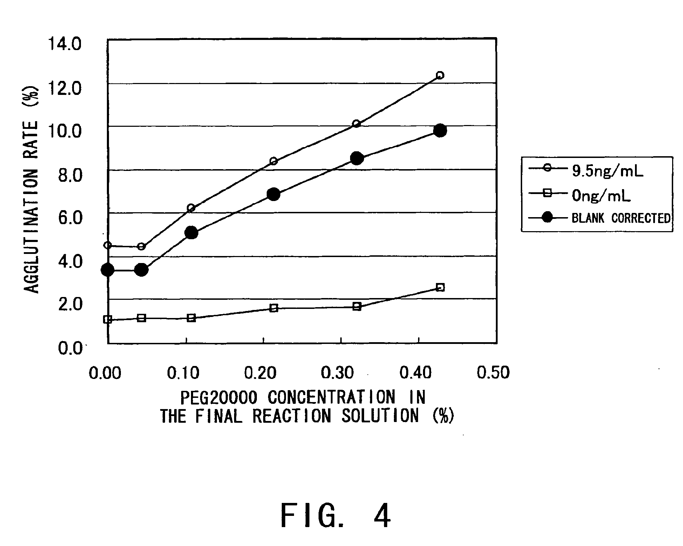

Fig. 4 is a diagram showing the results of measurement (in relation to reaction accelerator) obtained by performing the measurement method of the present invention using a measurement device with the configuration of Fig. 1. The plots in the diagram respectively represent the following results:

open squares, amount of antigen 0 ng/ml (blank value);
open circles, amount of antigen 9.5 ng/ml; and
closed circles, blank correction (9.5 to 0 ng/mL).

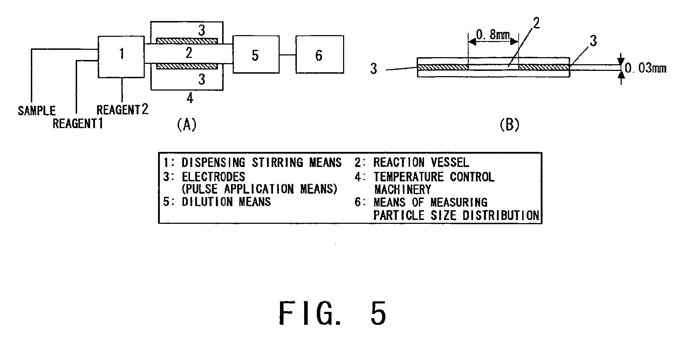

Fig. 5 (A) is a diagram illustrating the configuration of a device based on the present invention and (B) is a diagram illustrating a cross-sectional view of a pulse application vessel that composes a device based on the present invention. Numerals in the drawings indicate elements described in the explanation of numerals.

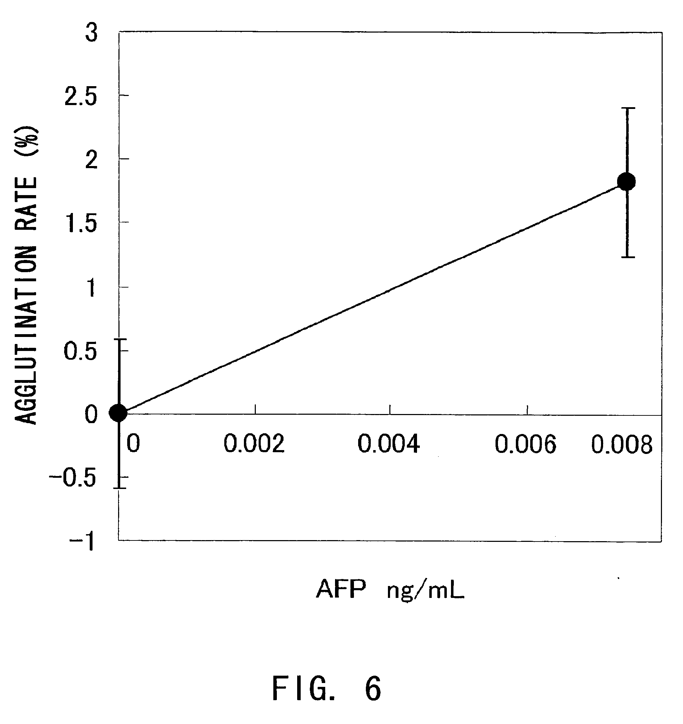

Fig. 6 is a diagram showing the results of measurement obtained by performing the measurement method of the present invention using a measurement device with the configuration of Fig. 5, wherein the reaction solution was maintained at low temperature during voltage pulse application. In the diagram, the vertical axis and the horizontal axis represent percent agglutination (%) and AFP concentration (ng/mL), respectively.

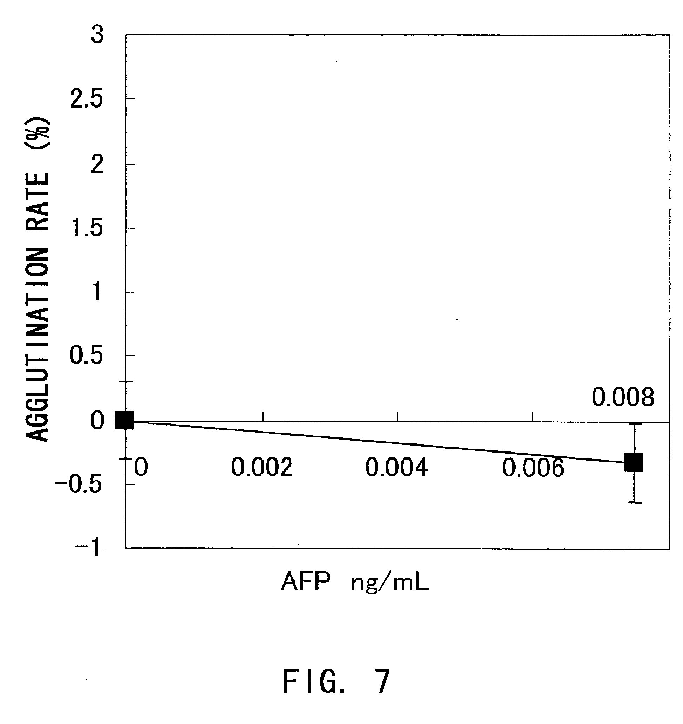

Fig. 7 is a diagram showing the results of measurement obtained by the control method of Fig. 6 (reaction solution without temperature control during voltage pulse application: room temperature). In the diagram, the vertical axis and the horizontal axis represent percent agglutination (%) and AFP concentration (ng/mL), respectively.

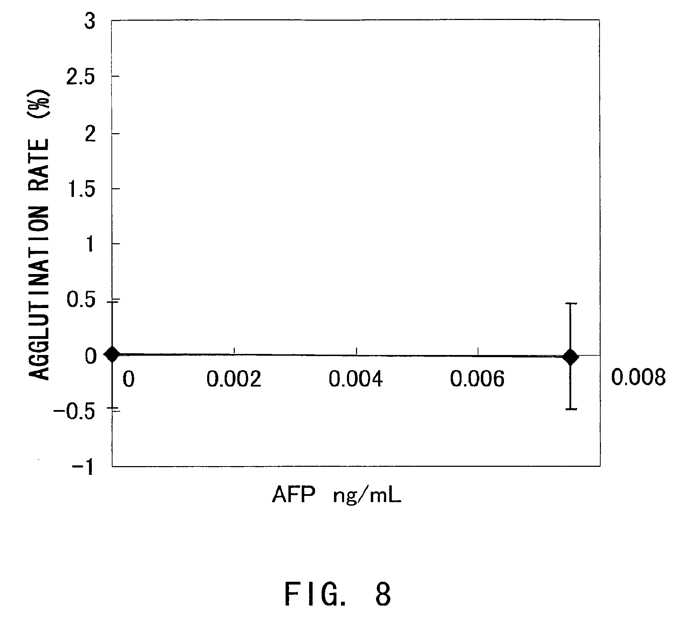

Fig. 8 is a diagram showing the results of measurement obtained by the control method of Fig. 6 (incubated at 37°C for 20 minutes with no voltage pulse being applied). In the diagram, the vertical axis and the horizontal axis represent percent agglutination (%) and AFP concentration (ng/mL), respectively.

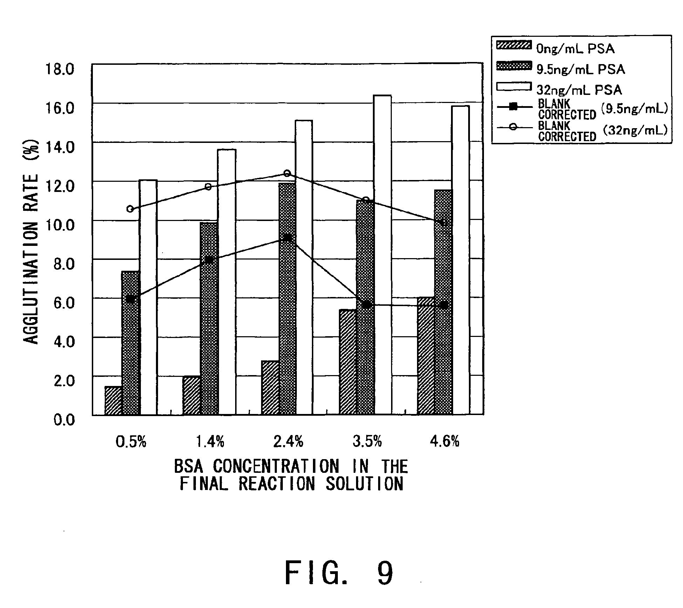

Fig. 9 is a graph showing effects on the percent agglutination of carrier particles of the bovine serum albumin (BSA) added to a reaction solution in measurement of a prostate-specific antigen (PSA) according to the present invention. In the diagram, the vertical axis and the horizontal axis represent percent agglutination (%) and final BSA concentration in the reaction solution, respectively. The columns show the results of PSA, from left to right, at 0 ng/mL, 9.5 ng/mL, and 32 ng/mL. Also shown in the graph are differences in the percent agglutination with PSA at 0 ng/mL and 9.5 ng/mL (closed squares) as well as differences in the percent agglutination with PSA at 0 ng/mL and 32 ng/mL (open circles) for each BSA concentration.

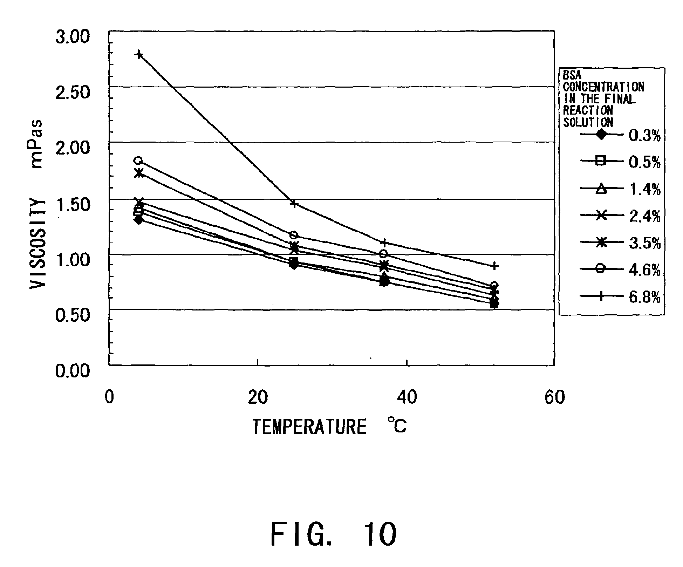

Fig. 10 is a graph showing effects of the bovine serum albumin (BSA) concentration and temperature of the reaction solution on the viscosity of the reaction solution. In the diagram, the vertical axis and the horizontal axis represent viscosity (mPas) and temperature (°C), respectively.

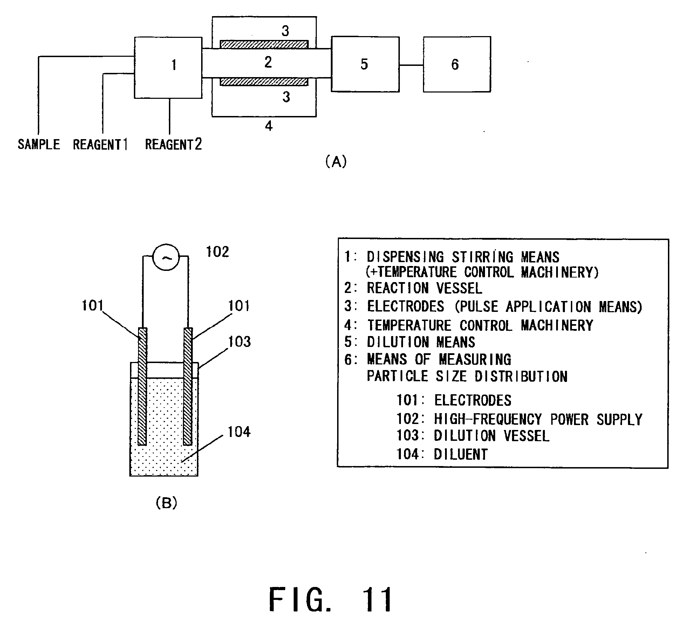

Fig. 11 (A) is a schematic view illustrating a device for measuring agglutination of carriers and Fig. 11 (B) is a schematic view illustrating dilution vessel 5 of Fig. 11 (A).

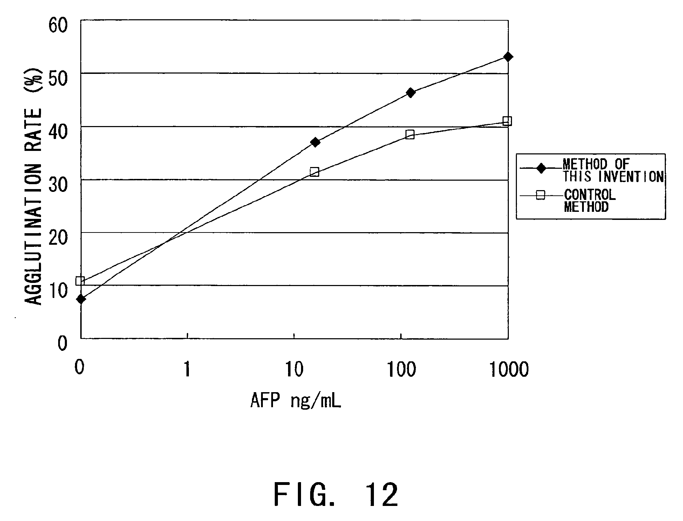

Fig. 12 is a graph showing percent agglutination of carriers when the reaction is conducted using AFP control serum as a sample solution and an anti-AFP antibody-sensitized latex reagent as a carrier. The percent agglutination in the case of dilution by voltage application is shown as closed rhombuses and the percent agglutination in the case of dilution without voltage application is shown as open squares. The vertical axis and the horizontal axis represent percent agglutination and AFP concentration, respectively.

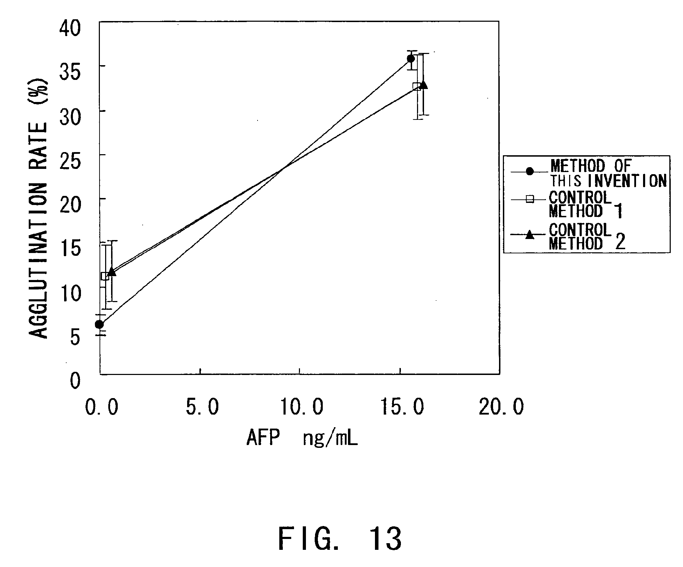

Fig. 13 is a graph showing percent agglutination of carriers when the reaction is conducted using AFP control serum as a sample solution and an anti-AFP antibody-sensitized latex reagent as a carrier. The percent agglutination in the case of dilution by voltage application is shown as closed circles, the percent agglutination in the case of dilution without voltage application is shown as open squares, and the percent agglutination in the case of dilution using a diluent to which voltage has been preliminarily applied is shown as closed triangles. The vertical axis and the horizontal axis represent percent agglutination and AFP concentration, respectively.

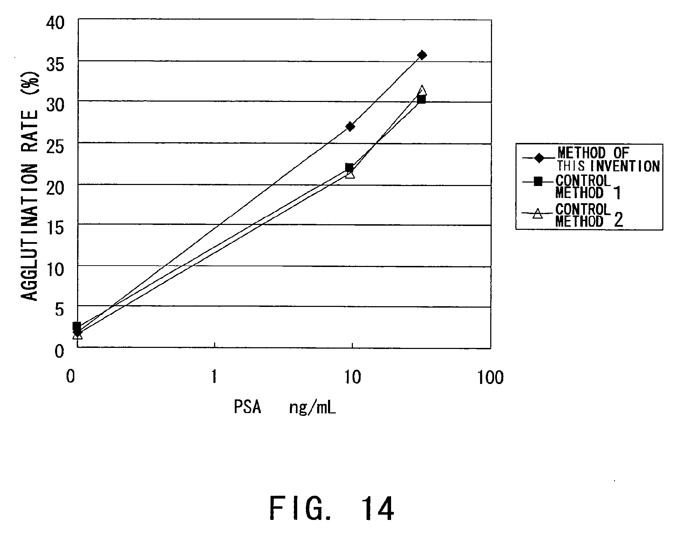

Fig. 14 is a graph showing percent agglutination of carriers in a reaction conducted by adding a reaction acceleration reagent, and using PSA control serum as a sample solution and anti-PSA antibody-sensitized latex reagent as a carrier. The percent agglutination in the case of dilution by voltage application is shown as closed rhombuses, the percent agglutination in the case of dilution without voltage application is shown as closed squares and the percent agglutination in the case of dilution using a diluent to which voltage has been preliminarily applied is shown as open triangles. The vertical axis and the horizontal axis represent percent agglutination and PSA concentration, respectively.

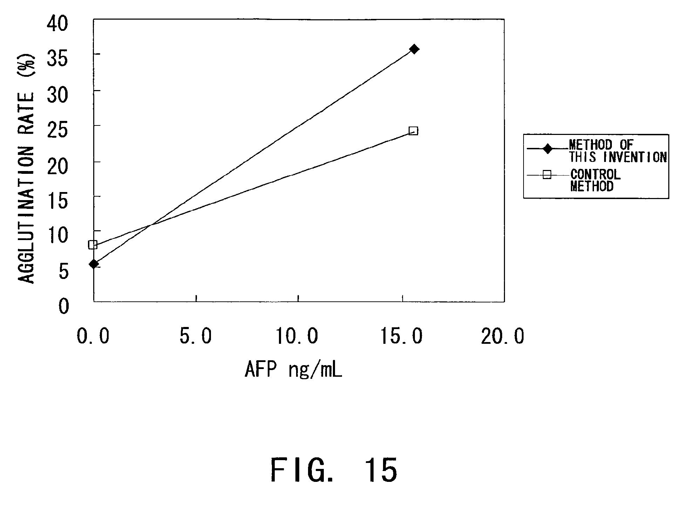

Fig. 15 is a graph showing the percent agglutination of carriers when 2.0 $\mu$m of an anti-AFP antibody-sensitized latex reagent was reacted as a carrier with AFP control serum. The percent agglutination in the case of dilution by voltage application is shown as closed rhombuses and the percent agglutination in the case of dilution without voltage application is shown as open squares. The vertical axis and the horizontal axis represent percent agglutination and AFP concentration, respectively.

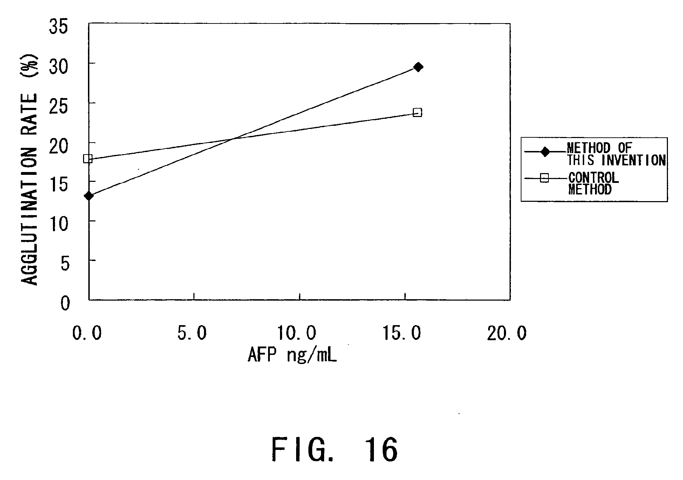

Fig. 16 is a graph showing the percent agglutination of carriers when 3 $\mu$m of an anti-AFP antibody-sensitized latex reagent was reacted with AFP control serum as a carrier. The percent agglutination in the case of dilution by voltage application is shown as closed rhombuses and the percent agglutination in the case of dilution without voltage application is shown as open squares. The vertical axis and the horizontal axis represent percent agglutination and AFP concentration, respectively.

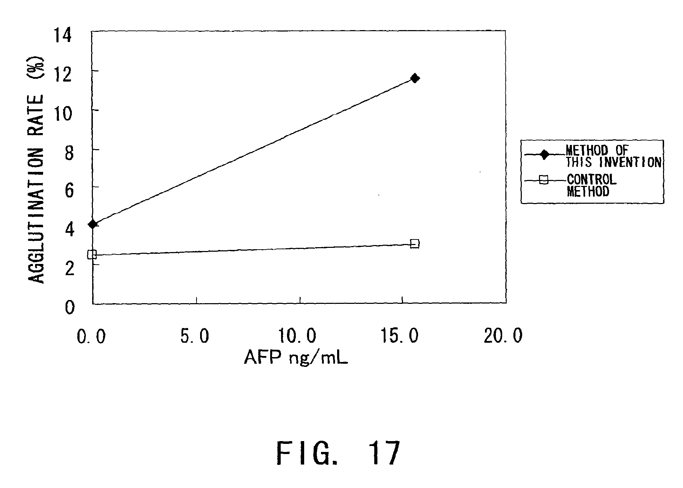

Fig. 17 is a graph showing the percent agglutination of carriers when 4.5 $\mu$m of an anti-AFP antibody-sensitized latex reagent was reacted with AFP control serum as a carrier. The percent agglutination in the case of dilution by voltage application is shown as closed rhombuses and the percent agglutination in the case of dilution without voltage application is shown as open squares. The vertical axis and the horizontal axis represent percent agglutination and AFP concentration, respectively.

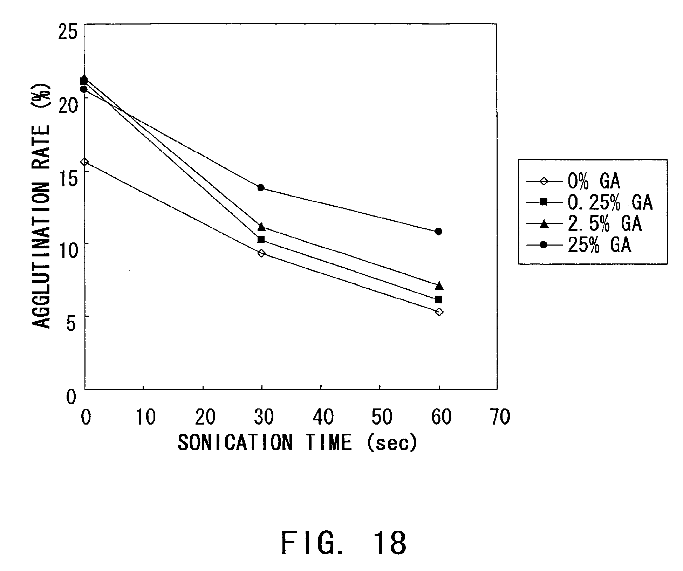

Fig. 18 is a graph showing the percent agglutination of carriers when AFP control serum was reacted with an anti-AFP antibody-sensitized latex reagent followed by sonication in which 0.25%, 2.5%, or 25% glutaraldehyde, or no glutaraldehyde was added. The vertical axis and the horizontal axis represent percent agglutination and duration of sonication, respectively.

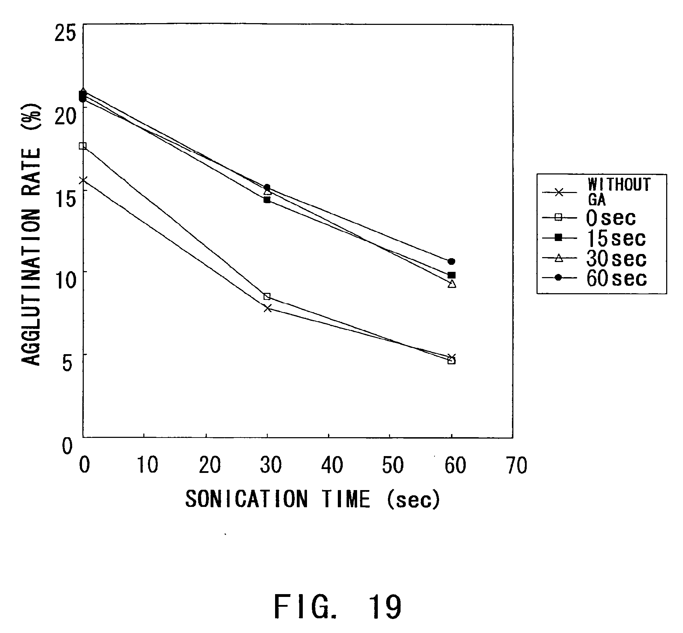

Fig. 19 is a graph showing the percent agglutination of carriers when AFP control serum was reacted with an anti-AFP antibody-sensitized latex reagent and incubated for 0, 15, 30, or 60 seconds with and without addition of glutaraldehyde, followed by sonication. The vertical axis and the horizontal axis represent percent agglutination and duration of sonication, respectively.

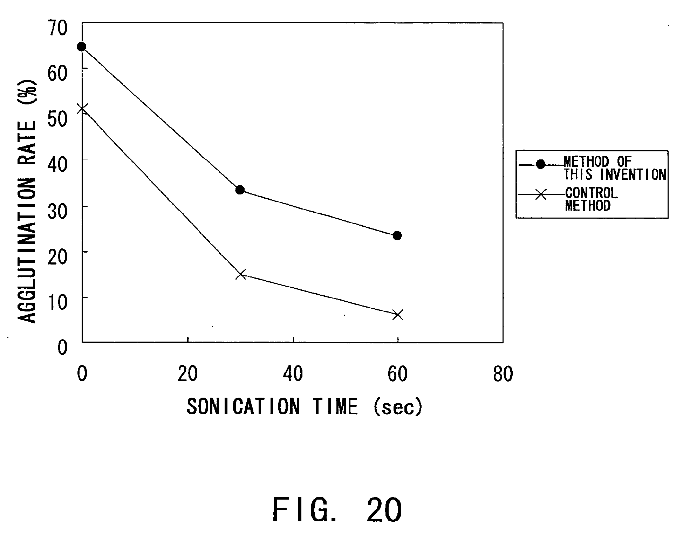

Fig. 20 is a graph showing the percent agglutination of carriers when 2.0 $\mu$m of an anti-AFP antibody-sensitized latex reagent was reacted with AFP control serum as a carrier and incubated with and without addition of glutaraldehyde, followed by sonication. The vertical axis and the horizontal axis represent percent agglutination and duration of sonication, respectively.

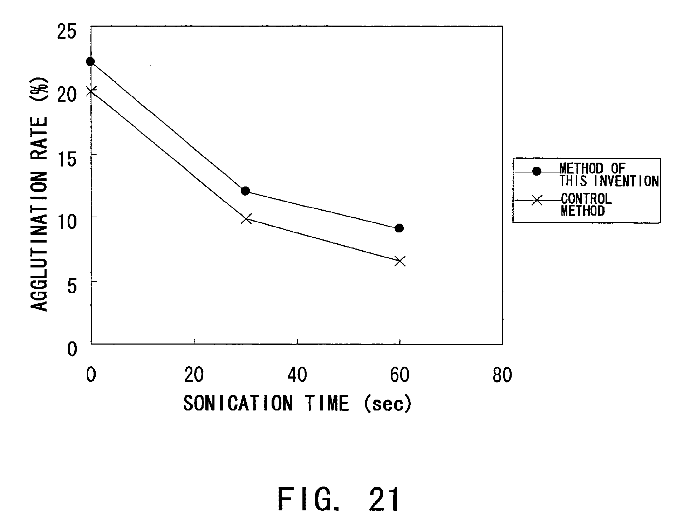

Fig. 21 is a graph showing the percent agglutination of carriers when 2.8 $\mu$m of an anti-AFP antibody-sensitized latex reagent was reacted with AFP control serum as a carrier and incubated with and without addition of glutaraldehyde, followed by sonication. The vertical axis and the horizontal axis represent percent agglutination and duration of sonication, respectively.

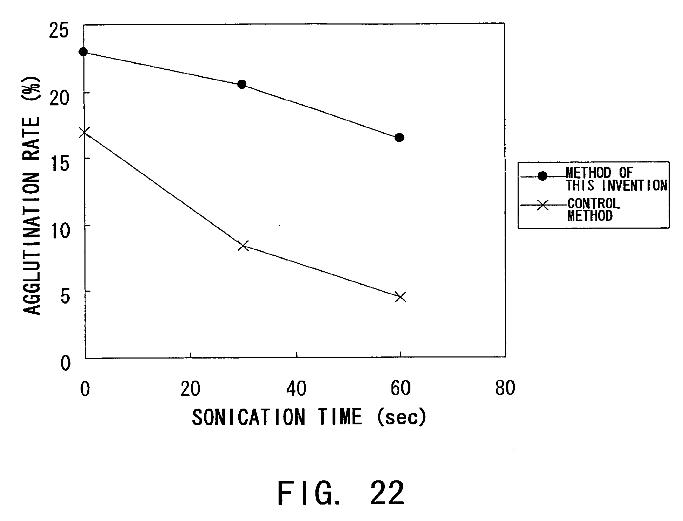

Fig. 22 is a graph showing the percent agglutinations of carriers when 1.7 $\mu$m of an anti-AFP antibody-sensitized latex reagent was reacted with AFP control serum as a carrier and incubated with and without addition of glutaraldehyde, followed by sonication. The vertical axis and the horizontal axis represent percent agglutination and duration of sonication, respectively.

Best Mode for Carrying Out the Invention

The present invention relates to methods for measuring affinity substances, comprising the steps of:

(1) incubating a mixed reaction solution comprising an affinity substance to be measured and carrier particles that are bound to a binding partner having an activity to bind to the affinity substance to be measured;

(2) applying voltage pulses to the reaction solution of step (1);

(3) after step (2), counting agglutinates of carrier particles formed by binding to the affinity substance to be measured, or unagglutinated carrier particles which have not bound to the affinity substance to be measured, or both; and

(4) after step (3), determining the level of the substance to be measured based on either or both of the level of agglutinate formation and the level of unagglutinated carrier particles.

The present invention comprises incubating a mixed reaction solution of carrier particles that are bound to a binding partner having an activity to bind to the affinity substance to be measured and the affinity substance to be measured before applying voltage pulses. The inventors have discovered that formation of agglutinates after application of voltage pulses is accelerated by incubating a reaction solution before application of voltage pulses. Namely, the reaction is accelerated by the incubation prior to application of voltage pulses.

In the present invention, incubation of a reaction solution is carried out, for example, at a temperature above room temperature. It is desirable that the incubation temperature is as high as possible as long as activities of the various reactive components contained in the reaction solution can be maintained. The incubation time is not limited. Namely, incubation can be carried out at an incubation temperature that does not cause modifications to the reactive components. The longer the incubation time, the further the acceleration effect will be enhanced. It is therefore desirable to preliminarily set the conditions of temperature and time in which the necessary level of acceleration effect can be expected.

Specifically, examples of the temperature condition for incubation are typically 37 to 90°C, preferably 40 to 90°C or 45 to 80°C. For example, a protein antigen as an affinity substance can be measured using an antibody as a binding partner according to the present invention. Antibody- or antigen-composing proteins are known to denature at high temperature. For example, many proteins do not denature at incubation conditions of 30 minutes at 56°C, which are conditions generally used for immobilizing serum. In addition, the inventors have observed that protein denaturation is negligible even at a temperature as high as 90°C in conditions of low protein concentrations such as in an immunoassay if the treatment is for a short period of time. For example, it has been observed that incubation for five to 180 seconds in the range of 45 to 80°C yields approximately the same level of reaction-accelerating effect (Fig. 3). Thus, the preferable incubation conditions of the present invention include five seconds or more, for example, five to 30 seconds at preferably 45 to 80°C, more preferably at 50 to 65°C.

It is needless to say that the present invention also includes longer incubation time. When reduction of incubation time is desired, a short period of five seconds or more may be adopted and the desired reaction-accelerating effect can be expected without sacrificing the reaction time. Also, at high-temperature conditions above 80°C, use of an incubation period of five to 180 seconds can avoid protein denaturation.

Alternatively, high-temperature condition will not become a problem when the method of the present invention is applied to reactions of heat-resistant substances. DNA, for example, is highly stable under high-temperature conditions. In cases where binding between DNAs is sought to be measured based on agglutination of carrier particles, higher temperatures may be chosen for the incubation temperature.

In the present invention, the mechanism in which a reaction is accelerated by incubation can be explained as follows. Carrier particles that align as a result of voltage pulse application form agglutinates through crosslinking between binding partners immobilized thereon and binding partners on other carrier particles via affinity substances. The series of reactions are thought to take place when the carrier particles become aligned. The findings attained by the inventors confirmed that incubation prior to application of voltage pulses contributes to the reaction efficiency. It was also revealed that, during such incubation, the binding partner on carrier particles becomes bound to the affinity substance. In fact, it is believed that the reaction can be made more efficient by allowing the binding partner to capture the affinity substance prior to alignment of carrier particles by applying voltage pulses.

Carrier particles are supposed to have a far more degree of freedom under conditions in which voltage pulses are not applied, as compared with when voltage pulses are applied. A binding partner on a carrier particle is therefore capable of making contact with an affinity substance so that the binding partner can capture the affinity substance. Upon application of voltage pulses, carrier particles with a binding partner that has captured the affinity substance will be aligned across the electric field along with other carrier particles. Since the binding partner has already captured the affinity substance, agglutinates are quickly formed via binding with the binding partner of other nearby carrier particles. When an electric field is intermittently applied, chances of making contacts will increase through repetition of alignment and dispersion of carrier particles, and formation of agglutinates will be accelerated.

In methods of detecting agglutination of carrier particles following the application of voltage pulses and measuring affinity substance by using the agglutination as an indicator, agglutinates of carrier particles can be said to form via the following primary and secondary reactions. Primary reaction: A reaction in which a binding partner on carrier particle captures an affinity substance. The crosslink structure between carrier particles via the affinity substance does not necessarily have to be formed yet. Secondary reaction: A reaction in which the binding partner on multiple carrier particles binds to an affinity substance. As a result, a crosslink structure (that is, agglutinate) is formed between carrier particles via the affinity substance.

The secondary reaction is accelerated by application of voltage pulses. Up until now, however, no specific conditions for accelerating the primary reaction have been revealed. The present invention can therefore be considered to provide conditions for accelerating the primary reaction. Namely, the incubation step before application of voltage pulses in the present invention can be said to be a step for accelerating the primary reaction.

In the present invention, water-soluble polymers can be added to a reaction solution before application of voltage pulses. By detecting the binding between an affinity substance and a binding partner based on particle agglutination reaction in the presence of the a water-soluble polymer, enhancement or stabilization of the agglutination reaction can be achieved. The concentration of water-soluble polymer in a reaction solution may be appropriately selected from, for example, 0.05 to 5%. The concentration is more preferably 0.1 to 3%, and even more preferably 0.3 to 1%. The possibility of nonspecific agglutination reaction tends to increase for compounds with high agglutination reaction-enhancing ability, at a concentration exceeding 5%. Also, at 0.05% concentration or lower, sufficient efficiency may not be expected.

As water-soluble polymers, polyethylene glycol, dextran, carboxymethylcellulose and such may be used. The molecular weight of polyethylene glycol is preferably 6,000 to 2,000,000. These water-soluble polymers may be used alone or in combination of two or more. To add a water-soluble polymer to a reaction solution, the required amount may be added beforehand to a carrier particle-containing reagent. Alternatively, the water-soluble polymer may be mixed as a reagent different from the carrier particle reagent. For example, the water-soluble polymer may be added to diluted sample solutions. It may also be added to multiple-reagent mixtures and diluents.

As a binary reagent system, a first reagent that contains a buffer solution or such, to be mixed with a second reagent containing carriers for measurement, is used. Nonspecific absorbents that absorb nonspecific substances such as heterophile antibodies, or substances that absorb rheumatoid factors may then be added to the first reagent.

Improvement in reaction efficiency by incubation before application of voltage pulses according to the present invention may be applied to agglutination-inhibiting reactions. More specifically, the present invention provides a method of measuring an affinity substance comprising the following steps. Incubation conditions are similar to those described above. Water-soluble polymers may also be added to solutions in agglutination inhibition systems.

(1') a step of incubating, before or after mixing with an agglutination reagent component, a reaction solution comprising an affinity substance to be measured and carrier particles that are bound to a binding partner with at least the activity to bind to the affinity substance to be measured, wherein the carrier particles agglutinate via the agglutination reagent and the agglutination is prevented by the affinity substance to be measured;

(2') a step of applying voltage pulses to the reaction solution of step (1') in the presence of the agglutination reagent component;

(3') after step (2'), a step of counting carrier particle agglutinates formed by binding to the agglutination reagent, or carrier particles whose agglutination is inhibited from by binding to the affinity substance to be measured, or both; and

(4') after step (3), a step of determining the level of the substance to be measured based on either or both of the level of agglutinate formation and the level of unagglutinated carrier particles.

As described above, the incubation of a reaction solution before application of voltage pulses is effective for facilitating agglutination reaction of carrier particles. It has already been mentioned that higher the incubation temperature, greater the effect. Meanwhile, the temperature of a reaction solution to which voltage pulses are applied will increase by Joule heat. When an electric current flows through a conductor, the heat generated at the conductor is Joule heat. The present inventors have found that the high-temperature condition during incubation affects agglutination reaction in an accelerating manner while a rise in temperature at the time of voltage pulse application affects agglutination reaction in an inhibitory manner. It is therefore advantageous to keep the temperature of the reaction solution low at the time of voltage application.

More specifically, the present invention provides methods for agglutinating carrier particles, comprising the step of applying voltage pulses to a reaction solution which contains a particular substance and carrier particles bound to a binding partner having the activity to bind to the particular substance, comprising maintaining the temperature of the reaction solution at 0°C to 20°C during voltage application.

The methods of the present invention can be utilized, for example, as methods for measuring an affinity substance using agglutination of carrier particles as an indicator. More specifically, methods for measuring an affinity substance are provided, comprising the steps of:

(1) applying voltage pulses to a mixed reaction solution comprising the affinity substance to be measured and carrier particles that are bound to a binding partner having the activity to bind to the affinity substance to be measured under the condition of 0°C to 20°C;

(2) counting, after step (1), agglutinates of carrier particles formed by binding to the affinity substance to be measured, or unagglutinated carrier particles which have not bound to the affinity substance to be measured, or both; and
(3) determining, after step (2), the level of the substance to be measured based on either or both of the level of agglutinate formation and the level of unagglutinated carrier particles.

Alternatively, the present invention provides methods for measuring an affinity substance, comprising the steps of:

(1') applying voltage pulses to a reaction solution comprising an affinity substance to be measured, carrier particles that are bound to a binding partner having the activity to bind to at least the affinity substance to be measured, and an agglutination reagent component under the condition of 0°C to 20°C;
(2') counting, after step (1'), agglutinates of carrier particles formed by binding to the agglutination reagent, or carrier particles whose agglutination is inhibited upon binding to the affinity substance to be measured, or both; and
(3') determining, after step (2'), the level of the substance to be measured based on either or both of the level of agglutinate formation and the level of unagglutinated carrier particles.

In the present invention, the temperature during voltage pulse application is typically 0 to 20°C, for example 0 to 15°C, preferably 1 to 8°C or 2 to 4°C. The temperature of the reaction solution will increase by the application of voltage pulses. To maintain the temperature of the reaction solution low, therefore, a cooling means may be advantageously utilized. Suitable cooling means for creating a local low-temperature environment include the Peltier element, for example. The Peltier element is an electronic element composed of semiconductor that utilizes the Peltier effect discovered by Jean Charles A. Peltier. When a direct current flows through an N-type semiconductor and a P-type semiconductor, temperature will be absorbed at one of the semiconductors while heat radiation will occur at the other semiconductor (heat exchange phenomenon). The temperature at the side of heat absorption lowers, so that cooling may occur. Commercially available Peltier elements are generally capable of cooling down to around -10°C. The cooling capacity of the Peltier element can freely be controlled by the electric current supplied to semiconductors. Therefore, during the time when voltage pulses are applied, the temperature may be monitored by a temperature sensor and a Peltier element may be operated as necessary to maintain the temperature of a reaction solution within the predetermined range.

Alternatively, if the reaction solution is sufficiently cooled at the time of voltage pulse application and the temperature of the reaction solution is still within the predetermined range after the application of voltage pulses, cooling at the time of voltage pulse application may not always be necessary. For example, if the temperature of the reaction solution at the end of voltage pulse application is 20°C or lower, the temperature requirement can be satisfied without cooling during application. Positive cooling during voltage pulse application is not mandatory when the reaction solution is sufficiently cooled beforehand, and in addition, a rise in the temperature of the environment where the reaction solution is placed may be repressed. In the present invention, the reaction solution to which voltage pulses are applied may be incubated beforehand. The incubation conditions are as mentioned before. If the reaction solution is incubated at a high temperature of 37 to 90°C, it must be sufficiently cooled before voltage pulse application. Typically, the volume of a reaction solution is 1 mL or less and therefore, the reaction solution can be cooled in an extremely short period of time. Conditions in which a reaction solution has been incubated at a high temperature is cooled before voltage pulse application at 0 to 20°C are preferable conditions of the present invention.

The mechanism in which a rise in temperature during voltage pulse application affects the agglutination reaction in an inhibitory manner may be considered as follows. In a reaction solution to which voltage pulses are applied, alignment and dispersion of carrier particles repeatedly occur. Dispersion of carrier particles is effective for increasing the chance of a binding partner on carrier particles making contact with an affinity substance (or an agglutination reagent component) in a reaction solution. At the same time, alignment of carrier particles is effective for crosslinking multiple carrier particles and forming agglutinates by binding to an affinity substance (or an agglutination reagent component). When the motion of carrier particles in a reaction solution is intensive, the carrier particles however may not be sufficiently aligned. A condition under which the temperature of a reaction solution has increased may be considered as a condition under which the Brownian motion of carrier particles contained in the reaction solution becomes intensive, so that alignment of the carrier particles at the time of voltage application becomes difficult. As a result, alignment of carrier particles by voltage application is inhibited, and the agglutination reaction is inhibited. When the temperature of a reaction solution is controlled at the time of voltage application according to the present invention, sufficient effects of carrier particle alignment can be obtained by voltage application, so that inhibition of agglutination reaction as a result of temperature rise may be repressed.

Increasing the viscosity of a reaction solution is also effective for repressing the motion of carrier particles in a reaction solution to which voltage pulses are applied. A typical reaction solution for agglutination reaction has a viscosity of less than 0.75 mPas. With such viscosity, the motion of carrier particles may not be repressed, and the agglutination reaction may be inhibited. On the contrary, the present inventors confirmed that, at a viscosity of 0.8 mPas or higher, the agglutination reaction may proceed efficiently. More specifically, the present invention provides methods for agglutinating carrier particles, comprising the step of applying voltage pulses to a reaction solution, which contains a particular substance and carrier particles bound to a binding partner that has the activity to bind to the particular substance, wherein the viscosity of the reaction solution is maintained at 0.8 mPas or higher during voltage application.

More specifically, the present invention provides methods for measuring an affinity substance, wherein the viscosity of a reaction solution is 0.8 mPas or higher, comprising the following steps:

(1) a step of applying voltage pulses to a mixed reaction solution comprising an affinity substance to be measured and carrier particles that are bound to a binding partner having the activity to bind to the affinity substance to be measured;
(2) after step (1), a step of counting agglutinates of carrier particles formed by binding to the affinity substance to be measured, or unagglutinated carrier particles which have not bound to the affinity substance to be measured, or both; and
(3) after step (2), a step of determining the level of the substance to be measured based on either or both of the level of agglutinate formation and the level of unagglutinated carrier particles.

Alternatively, the present invention provides methods for measuring an affinity substance, wherein the viscosity of a reaction solution is 0.8 mPas or higher comprising the following steps:

(1') a step of applying voltage pulses to a reaction solution comprising an affinity substance to be measured, carrier particles bound to a binding partner that has the activity to bind to at least the affinity substance to be measured, and an agglutination reagent component;
(2') after step (1'), a step of counting agglutinates of carrier particles formed by binding to the agglutination reagent, or carrier particles whose agglutination is inhibited by binding to the affinity substance to be measured, or both; and
(3') after step (2'), a step of determining the level of the substance to be measured based on either or both of the level of agglutinate formation and the level of unagglutinated carrier particles.

In the present invention, the viscosity of the reaction solution is typically 0.8 mPas or higher, for example, from 1 to 3 mPas, and preferably from 1 to 2 mPas. The viscosity of the reaction solution can be adjusted by adding compounds that are capable of adjusting viscosity. As compounds capable of adjusting viscosity, any compounds that do not interfere with the binding between an affinity compound and a binding partner may be utilized. For example, bovine serum albumin, casein, glycerin, sucrose, or choline chloride, may be added to increase the viscosity of a reaction solution. The amount of compound to be added may be appropriately selected, for example, from 0.05 to 5%, more preferably 0.1 to 3%, and even more preferably 0.3 to 1%. In addition, even if the composition of a reaction solution remains the same, the viscosity will generally increase when the temperature of the reaction solution lowers. Thus, application of voltage pulses under low-temperature condition is effective in terms of increasing the viscosity of a reaction solution.
Those skilled in the art can determine the appropriate amount to add, by adding these compounds to a reaction solution and then measuring its viscosity under the temperature condition for voltage pulse application. Methods for determining liquid viscosity are known. In general, rotational viscometers, ultrasonic viscometers, oscillational viscometers, and such are used.
Also, the present invention comprises methods for measuring an affinity substance, comprising the following steps (1) to (3) or (1') to (3') which further comprise, before step (2) or (2'), a step of diluting the reaction solution by a means for enhancing the binding between affinity substance and binding partner or the binding between agglutination reagent and binding partner.

(1) a step of applying voltage pulses to a reaction solution comprising an affinity substance to be measured and carrier particles that are bound to a binding partner having the activity to bind to the affinity substance to be measured;
(2) after step (1), a step of counting agglutinates of carrier particles formed by binding to the affinity substance to be measured, or unagglutinated carrier particles which have not bound to the affinity substance to be measured, or both; and
(3) after step (2), a step of determining the level of the substance to be measured based on either or both of the level of agglutinate formation and the level of unagglutinated carrier particles, or (1') a step of applying voltage pulses to a mixed reaction solution comprising an affinity substance to be measured, carrier particles that are bound to a binding partner having the activity to bind to the affinity substance to be measured, and an agglutination reagent component, wherein the carrier particles agglutinate via the agglutination reagent and the agglutination is inhibited by the affinity substance to be measured;

(2') after step (1'), a step of counting agglutinates of carrier particles formed by binding to the agglutination reagent, or carrier particles whose agglutination is inhibited by binding to the affinity substance to be measured, or both; and

(3') after step (2'), a step of determining the level of the substance to be measured based on either or both of the level of agglutinate formation and the level of unagglutinated carrier particles.

The dilution step of the present invention may be carried out before step (2) or (2') by any means of enhancing the binding between affinity substance and binding partner or the binding between agglutination reagent and binding partner. For example, a method of diluting the reaction solution under the conditions of voltage pulse application is a preferred dilution method of the present invention. More specifically, dilution is carried out by adding the reaction solution to a diluent to which voltage pulses have been applied. The diluent is placed between electrodes, across from which voltage pulses are applied. In other words, the diluent is placed between opposite electrodes.

In the dilution step of the present invention, the size of electrodes and the spacing between electrodes are not particularly limited as long as dilution is carried out under conditions in which voltage pulses are applied to the reaction solution. That is, the step features an initial contact between the reaction solution and the diluent after pearl chain formation occurs in an electric field across opposite electrodes. For example, in a dilution system shown in Fig. 6 (B), approximately the same effect is confirmed using electrodes of 2 to 12 mm in width, 10 to 50 mm in length and 0.01 to 0.04 mm in thickness, with a spacing of 5 to 20 mm between electrodes.

Also, voltage pulses are preferably those of an alternating voltage. Conditions for voltage pulse application can be any conditions that do not induce electrolysis of the reaction solution or the diluent. Voltage of the voltage pulses is, for example, 0.1 V to 1.2 V, and more preferably 0.3 to 0.9 V. Frequency of the voltage pulses is, for example, 2 KHz to 20 MHz, and more preferably 10 KHz to 500 KHz. The voltage pulses may assume any waveforms. Specifically, the voltage pulses may appear as square waves, sine waves, triangular waves, etc. More preferably, the voltage pulses are in square waves. In the present invention, it is desirable to apply voltage pulses during times including at least moments of contact between the reaction solution and the diluent. Effects of enhancing the binding in dilution can be expected even with only a very short period of application time. More specifically, the application time is 0.5 to 30 seconds, and typically one to ten seconds, for example, one to five seconds.

As a diluent, a salt-containing solution may be used. Specifically, a physiological saline solution, glycine buffer, phosphate buffer, or such to which a salt of 50 mM to 600 mM has been added may be used. Salts include sodium chloride, potassium chloride, calcium chloride. Diluents may contain preservatives, such as sodium azide, surface active agents, such as Triton X-100, glycerin, sucrose, etc.

In the present invention, the binding reaction between an affinity substance (or an agglutination reagent) and a binding partner which form an agglutinate is enhanced. The dipole moment effect of an electric field as a result of voltage pulses is thought to enhance the binding between them. In any case, it has been confirmed that dilution under the conditions of voltage pulse application represses disruption of agglutinates so that the dilution of a reaction solution may be achieved while maintaining the agglutinates.

Alternatively, as a dilution means capable of enhancing the binding between an affinity substance and a binding partner or the binding between an agglutination reagent and a binding partner, a binding enhancer capable of enhancing the binding between them may be utilized. Herein, a binding enhancer refers to a component capable of enhancing the binding between them when added to a reaction solution. For example, binding between proteins may be enhanced by adding compounds such as glutaraldehyde or carbodiimide. Immunological binding between a protein antigen and an antibody may therefore be enhanced by glutaraldehyde, carbodiimide, or such. These compounds are preferable binding enhancers of the present invention.

A binding enhancer acts on the functional groups of an affinity substance and a binding partner to chemically bind the two. Alternatively, in an agglutination-inhibiting reaction system, it enhances the binding between an agglutination reagent and a binding partner. As a result, an agglutinate formed by the binding of the two attains high physical stability. For example, when the affinity substance or agglutination reagent and the binding partner are proteins, they have intramolecular amino or carboxyl groups. These functional groups are crosslinked by chemicals such as glutaraldehyde and carbodiimide.

The concentrations of a binding enhancer in a reaction solution may be appropriately set according to the type of binding enhancer. More specifically, in the case of glutaraldehyde, for example, the final concentration in a reaction solution is typically from 0.1 to 25%, and preferably from 0.2 to 18%. The binding enhancer may be added to a reaction solution containing a conjugate of affinity substance and binding partner before diluting the reaction solution. The reaction solution to which a binding enhancer has been added can be diluted after incubation at 37°C for several seconds to about 20 seconds, preferably two to ten seconds, or two to five seconds. When glutaraldehyde or carbodiimide is used as a binding enhancer, the reaction solution may be diluted immediately after the addition.

Addition of a binding enhancer is effective as a dilution step of the present invention. In the present invention, a binding enhancer may be further combined in the dilution step under the previously described conditions of voltage pulse application. Specifically, a binding enhancer may be added to a reaction solution and then the reaction solution may be diluted under voltage pulse application according to the conditions described earlier. Alternatively, a diluent to which a binding enhancer has been added is utilized to dilute the reaction solution under voltage pulse application according to the conditions described earlier. The combination of the two can increase the effect of enhancing the binding.

Herein, diluting a reaction solution refers to reducing the concentration of carrier particles in a reaction solution by mixing the reaction solution with a diluent. The concentration of carrier particles in a solution is determined by the amount of sample and the amount of carrier particles supplied as a reagent. Also in the present invention, the concentration of carrier particles in a reaction solution is set within the range where agglutination of carrier particles can be accelerated through pearl chain formation. Specifically, the concentration of carrier particles in a reaction solution is typically 0.01 to 5% by weight, and more preferably 0.1 to 2% by weight. In relation thereto, dilutions of, for example, 100 fold or more, typically 1,000 fold or more, specifically 1,000 to 100,000 fold, and preferably 2,000 to 40,000 fold may be made. The concentration of carrier particles after dilution is $0.1 \times 10^{-5}$ to 0.005% by weight, and preferably 0.00001 to 0.001% by weight.

Herein, "affinity substance and binding partner having an activity to bind to the affinity substance" include every possible combination of substances that can participate in a binding reaction. Specifically, when one substance binds to another substance, one is the affinity substance and the other is the binding partner. The affinity substances and binding partners of the present invention may be natural substances or artificially synthesized compounds. The affinity substances and binding partners may be purified substances or substances containing impurities. Further, the affinity substances and binding partners may exist on cellular or viral surface.

Binding reactions between the affinity substances and binding partners of the present invention include, for example, the reactions listed below. Substances that participate in these reactions can either be an affinity substance or a binding partner of the present invention.

Reaction between an antibody and an antigen or a hapten (immunological reaction);
hybridization between nucleic acids having complementary nucleotide sequences;
reaction between a lectin and its receptor;
reaction between a lectin and a sugar chain;
reaction between a ligand and its receptor;
reaction between DNA and a transcription regulatory factor.

Among the above-listed binding reactions, a preferred binding reaction of the present invention can be, for example, an immunological reaction. Antigens participating in immunological reactions include the substances listed below.

Tumor markers:

AFP, CEA, CA19-9, PSA, etc.

Markers of the coagulation-fibrinolytic system:

protein C, protein S, antithrombin (AT) III, FDP, FDP-D-dimer, etc.

Infection markers:

CRP, ASO, HBs antigen, etc.

Hormones:

thyroid-stimulating hormone (TSH), prolactin, insulin, etc.

Tissue components:

myoglobin, myosin, hemoglobin, etc.

Others:

nucleic acids such as DNA.

These antigens include not only antigen molecules themselves but also fragments thereof, and those that are present on cell surface. These substances are only examples of antigenic substances and needless to say, the present invention is also applicable to other antigenic substances. For example, any antigenic substance that can be measured based on an immunological agglutination reaction using latex or blood cell as a carrier can be used as an affinity substance of the present invention.

Either an antigenic substance or an antibody recognizing the substance may be used as the affinity substance and the other as the binding partner. Herein, the affinity substance refers to a target substance to be measured. On the other hand, the binding partner refers to a substance that can be used as a probe to measure the affinity substance and has an activity to bind to the affinity substance. Thus, an antibody can be used as the binding partner when an antigen is measured. Conversely, an antibody recognizing an antigen can be used as the binding partner in the measurement of the antibody. For example, any antibody that can be measured based on an immunological agglutination reaction using latex or blood cell as a carrier can be used as an affinity substance of the present invention. Antibodies against HBs (surface antigen of hepatitis B virus), HBc (core antigen of hepatitis B virus), HCV (hepatitis C), HIV (AIDS virus), TP (syphilis), and such have been measured using immunological agglutination reactions.

Several reaction principles are known to use agglutination of carrier particles as an indicator for measuring the reaction between an affinity substance and a binding partner. Any of these reaction principles can be applied to the present invention. Examples of a measurement principle that uses agglutination of carrier particles as an indicator and applies the reaction between an affinity substance and a binding partner are described below.

Direct agglutination reaction:

The agglutination of carrier particles which results from the reaction between a target substance of measurement and its binding partner present on the carrier particles is detected. This principle is applicable, for example, to cases where an antigen molecule is measured using an antibody as the binding partner. Alternatively, the principle is also applicable when an antibody is measured as the affinity substance by using agglutination of antigen-bound carrier particles as an indicator. In general, the level of agglutination particle is directly proportional to the amount of affinity substance to be measured in a direct agglutination reaction. In general, the level of agglutination is directly proportional to the amount of affinity substance to be measured in a direct agglutination reaction. Specifically, the higher the level of agglutinate formation, the higher the level (namely concentration) of an affinity substance is. Conversely, when the level of unagglutinated carrier particles is high, the level (namely concentration) of an affinity substance is low.

Agglutination inhibition reaction:

A low-molecular-weight antigen called "hapten" hardly forms the antigen-mediated cross-linking structure required for the agglutination of carrier particles. Therefore, haptens cannot be detected based on the principle of direct agglutination reaction. In this case, it is possible to use the agglutination reaction that results from the binding of an antibody on carrier particles to a polyhapten that comprises two or more hapten molecules or fragments comprising the epitope. A polyhapten can crosslink two or more antibody molecules and agglutinate carrier particles. However, in the presence of a hapten, the reaction between a polyhapten and an antibody is inhibited and as a result, the agglutination of carrier particles is inhibited. The level of agglutination inhibition is directly proportional to the presence of hapten. Specifically, the amount of a target substance of measurement is inversely proportional to the level of agglutination reaction. Specifically, the level (i.e., concentration) of an affinity substance is low when the level of agglutinate formation is high. Conversely, the higher the level of unagglutinated carrier particles, the higher the level (i.e., concentration) of an affinity substance is.

Target antigens of measurement that are classified as haptens include the following components.

Hormones:

estrogen, estradiol

Drugs:

Theophylline.

In the present invention, measuring a hapten based on the principle of agglutination inhibition reaction requires a component that allows the agglutination of carrier particles bound to an anti-hapten antibody. Herein, a component that allows the agglutination of carrier particles bound to an anti-hapten antibody is referred to as an "agglutination reagent". An agglutination reagent is defined as a reagent that has specific affinity for an antibody as well as activity of crosslinking carrier particles via antibody binding. The polyhapten described above can be used as an agglutination reagent in hapten measurements.

In both the direct agglutination reaction and the agglutination inhibition reaction, a standard curve or regression equation may be prepared by measuring standard samples containing a predetermined concentration of affinity substance using the same reaction system, and measuring the level of agglutinates or unagglutinated carrier particles. The level of affinity substance in a sample can be determined either from the level of agglutinate formation or the level of unagglutinated carrier particles determined in a sample measurement, using the standard curve or regression equation.

The binding partners of the present invention are used to bind carrier particles. The carrier particles of the present invention include latex particle, kaolin, colloidal gold, erythrocyte, gelatin, liposome, and such. For the latex particle, those generally used in an agglutination reaction may be used. Polystyrene, polyvinyl toluene, and polymethacrylate latex particles are known. A preferred carrier particle is a polystyrene latex particle. It is possible to use latex particles that have surfaces onto which a functional group has been introduced through copolymerization of monomers having the functional group. Latex particles having a functional group, such as a carboxyl group -COOH, a hydroxyl group -OH, an amino group -$NH_2$, or a sulfone group -$SO_3$, are known. A binding partner can be chemically linked to latex particles having a functional group.

The mean particle diameter of a carrier particle may be, for example, in the range of 0.5 to 10 $\mu$m, more preferably in the range of 1 to 10 $\mu$m, most preferably in the range of 2 to 5 $\mu$m, when it is a latex particle. Smaller carrier particles may be used if they are oval particles showing strong dielectric polarization.

In contrast to the 0.05- to 0.6-$\mu$m carrier particles used in the conventional methods of latex agglutination turbidimetry, 1-$\mu$m or larger particles can be used in the methods of the present invention. Agglutination reaction is accelerated by using the step of applying voltage pulses. As a result, agglutination reaction proceeds adequately in a short time even when larger particles are used. Larger carrier particles have the benefits described below. First, apertures with a larger diameter size can be used for particle measurement and as a result, apertures are hardly clogged. In addition, larger carrier particles can be easily distinguished from the measurement-interfering substances in body fluids. Measurement accuracy is improved as a result. Meanwhile, by methods that use a counting means for capturing image information, device design for other counting means also becomes easier.

In the present invention, when the latex particles are used to replace other particles as carrier particles, particles that have a similar size as the latex particles may be utilized. For example, when particles such as kaolin, gold colloids, gelatin, or liposome are used as carrier particles, the carrier particles preferably have an average particle diameter of 0.3 to 20 $\mu$m.

A binding partner can be linked to particle carriers by methods suitable for the material. Those skilled in the art can appropriately select a method for linking the two. For example, latex particles can physically adsorb a protein such as an antigen, an antibody, or a fragment thereof. When latex particles have a functional group on their surface, a substituent that can be covalently linked to the functional group may be linked chemically. For example, an amino group -$NH_2$ in a protein can be linked to latex having a carboxyl group -COOH.

Carrier particles bound to a binding partner may be subjected to blocking treatment, if required. Specifically, the binding of non-specific proteins onto the surface of carrier particles can be prevented by treating the surface of carrier particles with an inactive protein. Bovine serum albumin, skimmed milk, or such can be used as an inactive protein. Furthermore, detergents or sugars may be added to the dispersion medium to improve the dispersibility of carrier particles. Alternatively, antimicrobial agents may be added to particle carriers to prevent the growth of microorganisms.

The present invention comprises the step of applying voltage pulses to a reaction solution containing an affinity substance and carrier particles that are bound to a binding partner. A method that applies voltage pulses to a reaction solution to perform an agglutination reaction is known (JP-A No. H7-83928). Carrier particles aligned along an electric field facilitate the binding reaction between the affinity substance and the binding partner on the carrier particles.

When the principle of agglutination inhibition reaction is applied, an affinity substance and carrier particles are aligned in the presence of an agglutination reagent. The agglutination reagent can be contacted after carrier particles have been contacted with an affinity substance to be measured. Alternatively, these three components can be contacted simultaneously by adding carrier particles to a premixture containing an affinity substance to be measured and an agglutination reagent. The affinity substance inhibits the reaction that forms agglutinates between the agglutination reagent and the binding partner.

An alternating current component or a direct current component can be used for the voltage pulse, and these two may be combined at one's choice. An alternating voltage is preferable in that it allows reaction solutions to undergo electrolysis easily. For an alternating voltage, square waves, rectangular waves, sine waves, or such can be used. The power supply frequency for an alternating voltage can be adjusted arbitrarily depending on the ionic strength of the reaction solution (reagent). An alternating voltage is applied to provide an electric field strength of 5-50 V/mm at its peak wave value. When the electric field strength is less than 5 V/mm, carriers can hardly form pearl chains and as a result, the acceleration of agglutination reaction becomes inadequate. When the electric field strength is greater than 50 V/mm, reaction solutions readily undergo electrolysis, making it difficult to measure agglutination reactions. More preferably, voltage is applied to provide an electric field strength of 10 to 20 V/mm. The alternating current frequency is preferably in the range of 10 KHz to 10 MHz, and more preferably in the range of 50 KHz to 1 MHz.

Herein, the voltage pulse typically refers to a voltage having a wave or waveform whose amplitude undergoes transitions from a steady state to a particular level, maintains the level for a finite time, and then returns to the original state. Alternating voltage is representative of such a voltage pulse. Alternating voltage is a periodic function of time with an average voltage value of zero. Alternating voltages include sine wave, rectangular wave, square wave, and sawtooth wave voltages, which have obvious periodic amplitudes. In general, the positive electric potential and the negative electric potential in an arbitrary cycle of alternating voltage have equal areas, making the sum of the two zero. Each area is defined by the curve above or below the horizontal axis, where the electric potential difference is zero. In the present invention, voltage pulses are applied to prevent electrolysis of reaction solutions. Accordingly, when electrolysis does not take place in a reaction solution, or if the electrophoresis, when actually occurs, can be suppressed to an extent that does not substantially interfere with the reaction, voltage pulses having a non-zero sum of positive and negative electric potentials may be applied.

Herein, the square wave or rectangular wave voltage pulse refers to a power supply that comprises cycles of positive electric potential/zero electric potential difference/negative electric potential and a constant voltage for at least either the positive or negative electric potential. The time interval between a state of zero electric potential difference and the succeeding zero state in square waves or rectangular waves is referred to as pulse width. Square wave refers to voltage pulses that form a nearly tetragonal shape when its voltage changes are drafted in a graph that has voltage on the vertical axis and time on the horizontal axis. The term "tetragonal" includes squares and rectangles. In contrast, rectangular waves are voltage pulses that have a rectangular shape, which does not include squares. Thus, square waves include rectangular waves. In the present invention, a generally preferred pulse width is 50 μsec or less, for example, in the range of 0.1 to 10 μsec.

There are no limitations on the duration of zero electric potential difference in square waves or rectangular waves. In general, the electric potential difference is zero at the moment of transition between positive and negative electric potentials. However, voltage pulses that maintain zero electric potential difference for a longer period may also be used in the present invention. For example, cycles of positive/negative electric potentials having a pulse width of 0.1 to 10 μsec may comprise a condition of zero electric potential difference that lasts 0.1 to 100 μsec.

In the present invention, the number of times that voltage pulses are applied to the reaction solution at step (1) or (1') is not limited. Namely, voltage pulses may be intermittently applied one or more times, for example, one to 20 times, typically one to ten times or one to five times. As a result of intermittent application, dispersion and alignment of carrier particles occur repeatedly. As a result, chances of a binding partner on carrier particles making contacts with an affinity substance or an agglutination reagent will increase. Namely, a reaction-accelerating effect may be expected from intermittent application of voltage pulses. In the present invention, when voltage pulses are applied multiple times, voltage pulses may be applied to a reaction solution from different directions. Specifically, voltage pulses may be applied to a reaction solution from different directions by disposing multiple pairs of electrodes in the reaction solution and switching the electrodes to which current is applied. Alternatively, electrodes in a reaction solution may be moved to change the directions of voltage pulses. Similar effects may be obtained by fixing the electrodes and moving the space which accommodates the reaction solution.

In addition, during application of multiple voltage pulses, carrier particles may be dispersed. Through the dispersion step, the effect of further increasing the chance of a binding partner making contact with an affinity substance or an agglutination reagent can be expected. Carrier particles can be dispersed during application of voltage pulses by agitating, shaking or giving vibration to a reaction solution.

In general, as the concentration of carrier particles in a reaction system becomes higher, pearl chain formation is enhanced and agglutination is accelerated. However, percent agglutination of carrier particles re-dispersed in the absence of a biologically specific reactive substance (background) tends to increase as the carrier particle concentration increases. In a known method that observes agglutinated particles based on two-dimensional information (JP-A No. 7-83928), the higher the carrier particle concentration, the higher the possibility that unagglutinated particles are mistaken as agglutinated particles. The particles are closer to each other as the particle concentration becomes higher, and thus it becomes difficult to distinguish particle agglutinates formed by agglutination from particles that are simply overlapping. Therefore, it is necessary to keep the particle concentration low in order to specifically distinguish agglutinates. Specifically, in the case of latex particles, the concentration of carrier particles in a reaction system, such as that disclosed in JP-A No. 7-83928, is preferably in the range of 0.01 to 1% W/W, more preferably in the range of 0.025 to 0.5% W/W, most preferably in the range of 0.05 to 0.1 % W/W. However, such particle concentrations are not necessarily the optimal conditions for pearl chain formation. That is, in agglutinate-counting methods that are based on two-dimensional information, specific identification of agglutinates is done at the sacrifice of particle concentration.

In the present invention, agglutinates can be specifically identified regardless of the particle concentration because measurement is based on the three-dimensional information of agglutinated particles. Thus, the present invention can provide optimal conditions for pearl chain formation. That is, the carrier particle concentration can be decided by taking into consideration the balance between a affinity substance to be measured and its binding partner having binding activity. Specific detection of agglutinates can be achieved even if a high carrier particle concentration is selected.

Usually, in the case of latex particles, the concentration of carrier particles in a reaction system in the present invention is preferably in the range of 0.01 to 5% W/W, and more preferably in the range of 0.1 to 2% W/W. This concentration range is two to ten times higher than that of two-dimensional information-based methods. The optimal carrier particle concentration can be appropriately adjusted depending on the carrier particle size, measurement sensitivity for the target affinity substance, and such.

In the present invention, salts may be added to a reaction solution to accelerate agglutination reaction. For example, a relatively high (10 mM or higher) concentration of salt may be added to accelerate agglutination reaction. However, a salt concentration of 600 mM or higher in a reaction system is unfavorable because such a higher concentration promotes electrolysis of the reaction solution. The salt concentration is more preferably in the range of 10 to 300 mM, most preferably in the range of 25 to 150 mM. When there is a possibility that a biological sample itself might contain a salt that accelerates agglutination reaction, the reagent's salt concentration may be adjusted so that the final salt concentration in a reaction solution falls within the range shown above. When direct-current voltage pulses are used, electrolysis takes place in a reaction solution even at a salt concentration of about 6 mM. Therefore, it is difficult to measure the biologically specific agglutination reaction in the presence of a salt.

Salts of the present invention can be selected from those that accelerate biologically specific agglutination reactions. Such salts include but are not limited to, for example, sodium chloride, potassium chloride, sodium nitrate, potassium nitrate, and ammonium chloride. A preferred salt of the present invention gives 100 cm$^2$/($\Omega$·mol) or higher molar electric conductivity in a 10mM aqueous solution at 25°C. More specifically, such preferred salts include, for example, sodium chloride, potassium chloride, and ammonium chloride.

Voltage pulses are applied to a mixed reaction solution comprising an affinity substance to be measured and carrier particles bound to a binding partner having the activity to bind to the affinity substance to be measured, and pearl chains are formed. Agglutinates may be formed by a specific reaction and redispersion will not occur even if voltage pulses are terminated. When a relatively strong external force is physically applied; however, the agglutinates may be disrupted, and accurate measurement cannot be made. To stabilize agglutinates formed by this specific reaction, the present invention comprises further strengthening the functional groups of the proteins attached to the agglutinate-forming carrier particles by chemical bonding. In the present invention, the operation is preferably carried out a step before diluting the carrier particles.

In the present invention, there are no limitations on the type of sample that contains an affinity substance. Specifically, it is possible to use an arbitrary sample that contains a affinity substance to be measured. For example, blood samples, samples collected from parts of the pharynx or such, saliva, sputum, urine, and feces are representative of biological samples. Other biological materials collected from a living body can also be used as samples for measuring biological substances in the present invention. Furthermore, cultures that are obtained by culturing such biological samples can be used as samples of the present invention. The biological materials can be used as samples directly, or if required, after being processed. For example, the biological materials may be used as samples after treatment of fractionation, dilution, lysis, extraction, or such.

In the present invention, samples used for the measurement may be a stock solution or an automatically diluted solution. The dilution fold may be set arbitrarily. When several types of reagents are required for a reaction, they may be added successively.

Herein, reagents that constitute a second reagent include, for example, the following reagents.

Reagents that preliminarily decompose and/or absorb substances that cause nonspecific reactions may be used in the present invention. Such reagents can be used as reagents that comprise a nonspecific reaction-suppressing agent. In combination, reagents comprising a nonspecific reaction-suppressing agent and reagents comprising carrier particles constitute the first and the second reagents. Reagents comprising a nonspecific reaction-suppressing agent may be preliminarily mixed with a sample, for example. For example, conventionally known agents that suppress nonspecific reactions may be used.

Immunoassay reveals the presence of various substances that cause nonspecific reactions in a sample. For example, globulins, such as rheumatoid factor, may interfere with the immunological reactions that make up an immunoassay. Agents that suppress nonspecific reactions may be used to prevent the globulin interference of immunoassay. For example, nonspecific effects can be absorbed by globulin-recognizing antibodies. The rheumatoid factor is a globulin derived from IgG or IgM, and can therefore be absorbed using an anti-human IgG antibody or an anti-human IgM antibody. Methods that prevent interference by decomposing causative substances of nonspecific reactions are known. Specifically, it is known that the interfering effects of globulins can be suppressed by reducing globulins to decomposition. The reduction of globulins can be achieved using dithiothreitol, 2-mercaptoethanol, or such.

Alternatively, it is possible to combine two or more types of reagents comprising carrier particles that are bound to binding partners having different binding activities. Such constitution allows different types of target affinity substances of measurement to be measured at a time. Each reagent can be added separately. Alternatively, a sample can be mixed with two or more preliminarily mixed reagents.

It is preferable to mix sample with reagents before voltage application. The two may be physically mixed using a stirrer bar. Alternatively, the two may be mixed by an electric means. Examples of electric means include a method that comprises physically displacing the positions of carrier particles by intermittently applying voltage pulses in different directions.

Steps that make up the measurement method of the present invention are specifically described below. A reaction solution containing a sample mixed with the necessary components is transferred to a vessel equipped with electrodes and voltage pulses are applied. When a reaction solution is preliminarily incubated prior to the application of voltage pulses, the incubation is carried out at a stage before and/or after the transfer to the electrode-equipped vessel. Upon application of an electric field, carrier particles undergo dielectric polarization to electrostatically attract each other, so that they may be linearly aligned. This phenomenon is called pearl chain formation. Thereafter, upon termination of the electric field, the carrier particles that have been linearly aligned will re-disperse instantaneously. Meanwhile, once binding partners have been bound to each other via an affinity substance during pearl chain formation, the carrier particles will not re-disperse even after the electric field is terminated and will remain agglutinated. By measuring either or both of the agglutinates thus formed and the unagglutinated carrier particles, the presence of the affinity substance can be detected or measured.

The measurement methods of the present invention comprise counting carrier particle agglutinates formed upon binding of a affinity substance to be measured, or unagglutinated carrier particles which do not bind to the affinity substance, or both, as an indicator. In the present invention, the particles can be measured after electric field is removed. Alternatively, the particles in an electric field can be measured without the electric field being removed. For example, the particles in an electric field can be counted by removing them from the electric field. Further, the process of dispersing particles can be conducted before particles are counted. Particles that have agglutinated due to nonspecific factors can be dispersed in the dispersion process before counting. As a result, improvement of measurement accuracy can be expected. Particles can also be dispersed by stirring or diluting a reaction solution.

In order to count the agglutinated carrier particles, known methods may be utilized. For example, a method for determining the level of agglutination based on two-dimensional information is publicly known. Specifically, microscopic images of a reaction solution are scanned to count the number of either or both of the agglutinates and unagglutinated particles per unit area.

Alternatively, in the present invention, carrier particles may be counted using three-dimensional information as an indicator. Herein, counting using three-dimensional information as an indicator refers to counting particles and/or agglutinates based on the measurement results of three-dimensional information of the particles and/or agglutinates. Counting carrier particles based on three-dimensional information is a preferred method of counting in the present invention.

There is no limitation on the method of measuring three-dimensional information. Herein, "counting" refers to determining the number of particles and/or agglutinates. The number of particles and/or agglutinates can be determined by simple counting. Alternatively, agglutinated particles and unagglutinated particles can be counted separately. Furthermore, in measuring agglutinated particles, the number of agglutinates may be determined for each number of agglutinated particles. There are known methods for counting particles using three-dimensional information as an indicator.

Measurement methods that are based on physical principles can be advantageously applied as the particle-counting methods in the present invention. Herein, "physical measurement methods" refers to measurement methods that enable the evaluation of inherent physical information of particles or agglutinates. In other words, the inherent physical information of particles or agglutinates is a result of true measurement. On the other hand, methods that analyze two-dimensional information obtained from graphic information also detect non-agglutinated overlapping particles as agglutinates. Such detection results are not considered inherent physical information of particles.

The use of a flow system is advantageous when the particles or agglutinates are measured physically. A flow system is a system which is capable of analyzing physical information of particles that pass through a minute flow cell. Physical measurements can be achieved conveniently by using a flow system. Specifically, physical measurements in the present invention comprise the step of counting by using a flow system to measure the three-dimensional information of particles and/or agglutinates. Methods that use three-dimensional information as an indicator to physically count particles include, for example, the Coulter principle and laser diffraction/scattering methods.

The Coulter principle (USPA 2656508 in 1953) is an analysis method for determining the volume of a particle based on the change of electric resistance resulted from passing of the particle through an aperture (small hole), which has electrodes on both sides. When a minute electric current is allowed to pass through an electrolytic solution between two electrodes, particles that are suspended in the electrolytic solution are aspirated, passed through an aperture, and then replaced by an equivalent volume of electrolytic solution. As a result, the electric resistance between electrodes is altered. The particle number and size (volume) can be determined by measuring this change. The electrostatic capacity method is available as a method for measuring volume; however, most of the methods that are in practical use are electric resistance methods.

The aperture size can be appropriately adjusted to accommodate the subject particle of analysis. When agglutination of carrier particles such as those used in general immunological particle agglutination reactions is detected, the aperture size is typically in the range of 30 to 1000 $\mu$m, and preferably in the range of 50 to 200 $\mu$m.

It is advantageous to have an aperture size that is several to several hundred times greater, for example, several to a hundred times greater, preferably 5 to 50 times greater than the mean particle diameter of carrier particles. In this case, highly accurate and sensitive measurements can be realized by detection of signals proportional to the volume. The sensitivity is higher when the aperture size-to-particle diameter ratio is small. However, when the ratio is too small, particles tend to clog up the aperture; when the ratio is too large, sensitivity of particle detection decreases; both cases are unfavorable.

More specifically, when the carrier particles to be counted have a particle diameter of, for example, 1 to 5 $\mu$m, particularly 2 to 3 $\mu$m, the aperture size may be selected from a range of 30 to 100 $\mu$m, preferably 50 to 80 $\mu$m, for example, 65 to 75 $\mu$m. Carrier particles that have a size of 2 to 3 $\mu$m are particularly preferred in the methods for measuring affinity substances by the present invention.

Specifically, the present invention provides methods for measuring affinity substances, which comprise:

(1) a step of combining carrier particles having a mean particle diameter of 2 to 3 $\mu$m with a affinity substance to be measured and applying voltage pulses, wherein the carrier particles are bound to a binding partner having an activity to bind the affinity substance to be measured; or (1') a step of combining carrier particles having a mean particle diameter of 2 to 3 $\mu$m with a affinity substance to be measured and an agglutination reagent component, and applying voltage pulses, wherein the carrier particles are bound to a binding partner having an activity to bind the affinity substance to be measured, and wherein the affinity substance to be measured inhibits agglutination of the carrier particles by the agglutination reagent;

(2) a step of counting carrier particle agglutinates formed upon binding of the affinity substance to be measured, or unagglutinated carrier particles which do not bind to the affinity substance to be measured, or both, using their three-dimensional information as an indicator after step (1), wherein an aperture of size 50 to 80 $\mu$m according to the Coulter principle is used; or

(2') a step of counting carrier particle agglutinates formed upon binding of the agglutination reagent, or carrier particles of which agglutination is inhibited through binding of the affinity substance to be measured, using their three-dimensional information as an indicator after step (1'), wherein an aperture of size 50 to 80 $\mu$m according to the Coulter principle is used; and

(3) a step of determining the level of the target substance of measurement based on either or both of the level of agglutinate formation and the level of unagglutinated carrier particles after step (2) or (2').

In general, the smaller the aperture size, the more accurately unagglutinated particles can be counted. Conversely, greater aperture size reduces the chance of an aperture being clogged with agglutinated particles. Aperture clogging decreases analysis efficiency, which can be improved by reducing the clogging frequency. For example, if agglutinated particles are predicted to be formed in great numbers, aperture clogging can be prevented by setting the aperture size to be slightly larger. Alternatively, a similar effect can be expected by using carrier particles with a small particle diameter. Further, the proportion of agglutinated particles may be reduced by diluting the sample to thereby prevent aperture clogging. In general, appropriate conditions may be selected for each case depending on the expected detection sensitivity, the predicted concentration of target substance to be detected, and the device configuration (aperture size, in particular).

The proportion of agglutinated particles can be determined by counting agglutinated particles by the procedure described above. The "proportion of agglutinated particles" refers to the proportion of agglutinated particles among the total particles counted. The proportion of agglutinated particles is also referred to as "percent agglutination (agglutination rate)". Furthermore, percent agglutination is determined for standard samples with known analyte concentrations, and the relation between the two is plotted on a graph to produce a standard curve. The concentration of a affinity substance to be measured in a sample can be revealed by checking percent agglutination of the sample against the graph.

Alternatively, the above-mentioned standard curve can also be expressed as a regression equation. Once a regression equation is obtained, the concentration of a affinity substance to be measured can be calculated by substituting percent agglutination into the regression equation.

On the other hand, laser diffraction/scattering methods are used to count particles and measure their mean diameter by detecting fluctuations generated from laser irradiation of particles. In either case, for the purpose of improving measurement accuracy, it is preferable to dilute reaction particles, apply sonication, and/or use a sheath flow system, and such to prevent false measurements of particles.

Methods for measuring particle volume also include the methods described below.

Centrifugal sedimentation method: a method for determining particle diameter distribution by the Stokes equation, which represents the relation between particle sedimentation rate in a solution and particle diameter. Photocentrifugal sedimentation methods use a phenomenon based on Stokes' law: larger particles sediment faster than smaller ones when they have the same specific gravity. The particle concentration is analyzed as the change in turbidity from light transmission. The particle size distribution can be determined by the procedure described above.

Capillary system: Poiseuille flow is generated in a capillary when the viscous fluid that flows through the capillary has a low Reynolds number. Since this flow is faster at the center of the capillary and slower near the capillary wall, large particles travel in fluxes that are faster on average and smaller particles travel in fluxes that are slower on average. Briefly, particles traveling through a capillary of given length are size-separated and detected according to the differences of their moving velocities.

Three-dimensional image analysis: Three-dimensional particle information can be obtained by analyzing graphic information of two or more images taken from different angles. Alternatively, three-dimensional particle information can be obtained by scanning graphic information along the z axis in the xy plane.

In the measurement methods of the present invention, agglutinated (or unagglutinated) carrier particles are counted. The target affinity substance of the measurement is measured qualitatively or quantitatively based on the counting results. In such qualitative measurements, the presence of a affinity substance to be measured is indicated by the presence of agglutinated particles. Alternatively, detection of agglutination inhibition in an agglutination inhibition reaction proves the presence of the target of measurement.

Alternatively, in such quantitative measurements, the level of agglutination can be correlated with the amount of affinity substance to be measured. More specifically, samples containing a known concentration of affinity substance are measured preliminarily using the measurement methods of the present invention to unravel the relation between the amount of affinity substance and the result of agglutinated particle detection. Then, samples are measured by the same measurement procedure. The amount of affinity substance can be determined from the result of agglutinated particle detection based on volume. In the case of an agglutination inhibition reaction, quantitative measurements can also be achieved by the same procedure described above.

In methods for counting particles and/or agglutinates, formulae such as [number of particles that form agglutinates of two or more particles]/[total number of particles], or [number of single particles]/[total number of particles], can be selected as means for counting a specific number of particles according to the purpose. The total number of particles may be determined as the total number of particles measured within a fixed time period of measurement, or in a literal sense, the total number of particles in a reaction solution when the entire reaction solution is the target of analysis. When the total volume of a reaction solution is known, the total number of particles in a reaction solution can be estimated by counting a portion of the reaction solution.

Alternatively, the affinity substance can be detected or measured based on the number of particles and/or agglutinates detected during a certain period of time by an electric resistance method, laser diffraction/scattering method, or such. That is, the number of particles counted decreases with time because single particles agglutinate to form agglutinates in agglutination reactions. Alternatively, it is possible to use the time required for counting a specific number of particles and/or agglutinates as an indicator. When such counting methods are used in the present invention, the relation between the number of particles and/or agglutinates and the amount of affinity substance can be expressed in a regression equation. For particles that have been sensitized with an antibody, the proportion of agglutinates comprising two or more particles increases depending on the antigen concentration. In this case, percent agglutination represented by [number of particles forming agglutinates consisted of two or more particles]/[total number of particles] converges to 1.00 (100%).

When compared with methods that analyze two-dimensional graphic data, methods that measure three-dimensional particle information, whether it be the Coulter principle or a laser diffraction/scattering method, allow high-accuracy analyses even with a simple device configuration. As described above, the volume of reaction solution is restricted in analyses of two-dimensional graphic data. In contrast, there are no limitations on the reaction solution volume in methods that measure three-dimensional information using flow-based analytical techniques. In addition, there are no limitations on the physical geometry of reaction space. These reasons attribute to a simpler device configuration. The fact that the reaction solution volume can be set freely further contributes to the reproducibility and detection sensitivity.

The present invention applies to agglutination-inhibiting reaction systems. The following describes principles for the immunological particle agglutination reaction based on agglutination inhibition reactions that use agglutination reagents. The present invention can be applied to immunological particle agglutination reactions by using the steps described above. Steps consisting of applying voltage pulses and analyzing levels of agglutinate formation or levels of unagglutinated carrier particles can be achieved by the specifically described methods above.

When the present invention is implemented based on the principle of agglutination inhibition reaction, it is preferable to select conditions that allow a larger number of agglutinates comprising two or more particles to be formed. Alternatively, methods for evaluating the level of agglutination using [number of single particles]/[total number of particles] as an indicator are preferred. When the principle of agglutination inhibition reaction is applied, use of the above formula can be expected to provide a higher sensitivity than analyses based on the [number of particles forming agglutinates consisted of two or more particles]/[total number of particles] formula.

In addition, the present invention provides devices for carrying out the measurement methods described above.

Specifically, the present invention provides devices for agglutinating carrier particles, comprising a means for applying voltage pulses to a reaction solution which contains a particular substance and carrier particles bound to a binding partner having the activity to bind to the particular substance, wherein the devices have a means for heating the temperature of the reaction solution to 37°C to 90°C.

Alternatively, the present invention provides measurement devices for measuring the binding between an affinity substance to be measured and carrier particles bound to a binding partner having the activity to bind to the affinity substance to be measured, using the agglutination of carrier particles by the affinity substance or an agglutination reagent as an indicator, comprising the following elements.

1a: a space for retaining a reaction solution;
1b: a means for incubating the temperature of the reaction solution at 37°C to 90°C;
1c: a means for applying voltage pulses to the reaction solution;
1d: a means for maintaining the temperature of the reaction solution at 0°C to 20°C at the time of voltage pulse application; and
1e: a means for counting either or both of the carrier particles and agglutinates of carrier particles contained in the reaction solution.

Configuration examples of measurement devices of the present invention comprising the above elements are illustrated in Fig. 1 and Fig. 5.

Also, the present invention provides measurement devices for measuring the binding between an affinity substance to be measured and carrier particles bound to a binding partner having the activity to bind to the affinity substance to be measured, using the agglutination of carrier particles by the affinity substance or an agglutination reagent as an indicator, comprising the following elements.

2a: a space for retaining a reaction solution which contains a sample containing the affinity substance to be measured and carrier particles bound to a binding partner having the activity to bind to the affinity substance to be measured, or a reaction solution which further contains an agglutination reagent;
2b: a means for applying voltage pulses to the reaction solution;
2c: a means for diluting the reaction solution; and
2d: a means for counting either or both of the carrier particles and agglutinates of carrier particles contained in the reaction solution.

A configuration example of the measurement devices of the present invention comprising the above elements is illustrated in Fig. 11.

In the present invention, for elements 1a and 2a: space for retaining a reaction solution, any space for retaining a reaction solution may be utilized. It is advantageous to utilize a small-volume space for the reaction of a trace amount of a sample. For example, a space as small as 1 $\mu$L to 10 mL, and preferably 10 to 500 $\mu$L may be utilized. This space may also be equipped, as needed, with a means for supplying samples and reagents or a means for measuring carrier particles to be described later. A reaction solution to be accommodated by the space is composed of a sample containing the affinity substance to be measured and carrier particles bound to a binding partner having the activity to bind to the affinity substance to be measured. Alternatively, in an anti-agglutination reaction system, an agglutination reagent component is also added.

In the present invention, element 1a: space for retaining a reaction solution, is equipped with 1b: means for incubating the reaction solution at a temperature of 37°C to 90°C. In order to maintain the reaction solution at a predetermined temperature, for example, a temperature sensor and a means for heating may be utilized. As a means for heating, a heater or a Peltier element may be utilized.

Next, elements 1c and 2b in the present invention: means for applying voltage pulses to a reaction solution will be described. The voltage pulses are applied through electrodes that are in contact with the reaction solution. Electrodes for aligning the carrier particles across an electric field are utilized also in the prior art documents described earlier. These known electrodes can be utilized for the present invention. The devices of the present invention can be equipped with a power source for supplying the electrodes with voltages.

The electrodes for supplying voltage pulses in the device of the present invention are composed of at least one set of electrodes (two electrodes). In order to apply voltage pulses in multiple different directions, three or more electrodes may also be provided. For example, three electrodes A, B and C are disposed, so that voltage pulses may be applied in three directions A-B, B-C and A-C. Otherwise, two sets (four) of electrodes can also be arranged to apply orthogonal voltage pulses.

In addition, electrode-driving machinery can be provided to apply voltage pulses in different directions. For example, by rotating electrodes in a reaction solution, voltage pulses can be applied from multiple different directions.

Further, the devices of the present invention can be equipped with a means for agitating a reaction solution, a means for shaking a reaction solution or a means for vibrating a reaction solution. These means are all useful as means for dispersing carrier particles during multiple times of voltage pulse application.

The devices of the present invention have element 2c: means for diluting a reaction solution. In the present invention, a means for diluting a reaction solution may be referred to as a dilution means below. A dilution means is composed of machinery for retaining a diluent, sampling at least a portion of a reaction solution, and mixing it with the diluent. Also, a dilution means is composed of a space capable of retaining a diluent that yields the predetermined dilution ratio. Dilution ratio of the present invention is, for example 100 fold or more, typically 1000 fold or more, specifically 1,000 to 100,000 fold, and preferably 2,000 to 40,000 fold.

The dilution means of the present invention are preferably equipped with machinery capable of enhancing binding in agglutinate formation. Specifically, under the conditions of voltage pulse application, a dilution means capable of mixing a reaction solution with a diluent is a preferred dilution means of the present invention. For example, electrodes for applying voltage pulses to a diluent can be placed in the above-described diluent-retaining space. Alternatively, the dilution means of the present invention can comprise machinery for adding a binding enhancer to a reaction solution. Configurations for enhancing the binding for forming these agglutinates can be provided alone or in combination of both.

The devices of the present invention are equipped with 1d: means for maintaining the temperature of the reaction solution at 0°C to 20°C at the time of voltage pulse application. As a preferred means for maintaining the temperature of the reaction solution at 0°C to 20°C, a temperature sensor and a Peltier element can be used.

In addition, the devices of the present invention comprise elements 1e and 2d: means for counting either or both of carrier particles and agglutinates of carrier particles contained in a reaction solution. The means for counting may be provided in the space described above. Alternatively, counting can also be carried out after the reaction solution retained in the space is withdrawn from the space and introduced into the means for counting. Also, the means for counting comprises, for example, machinery for analyzing carrier particles contained in a diluted reaction solution. Alternatively, particles may also be counted after the diluted reaction solution is withdrawn from the space that retains the diluent and introduced into the means for counting. The carrier particles or agglutinates can be analyzed based on two-dimensional image information or three-dimensional physical information.

Measurement devices that apply the Coulter principle or a laser diffraction/scattering method can be used as means for counting agglutinated or unagglutinated carrier particles using three-dimensional information as an indicator. When the Coulter principle is used, for example, a reaction solution is transferred from the above-mentioned space to an aperture equipped with Coulter-principle electrodes to carry out the required analyses. The aperture size can be adjusted appropriately based on the criteria described above. It is possible to employ a structural body to switch between two or more apertures of different sizes, and use them according to the diameter of particles used as the reagent or the predicted proportion of agglutinated particles. The devices of the present invention may be equipped, for example, with a structural body that switches the flow path in order to transfer the reaction solution to multiple apertures. Furthermore, structural bodies that automatically select a flow path according to the reagent type, the predicted proportion of agglutinated particles, or such may be used in combination. Alternatively, the devices of the present invention may be equipped with a structural body that automatically adjusts the detection sensitivity according to the change in aperture size. The structural body for adjusting detection sensitivity includes, for example, those that analyze using a relatively larger aperture size first and switching to a smaller aperture when the proportion of agglutinated particles is predicted to be small. When a laser diffraction/scattering method is used, the analysis may be carried out by introducing the reaction solution into an optical cell for analysis by the same procedure described above. An analysis system for particles and/or agglutinates based on three-dimensional information as an indicator is preferable for a counting means in the present invention.

In the present invention, the carrier particles that form pearl chains in an electric field may be counted after being re-dispersed, if necessary. The device of the present invention may be equipped with a structural body for re-dispersing carrier particles. The carrier particles can be re-dispersed through dilution or sonication.

The above (1a) to (1e) or (2a) to (2d) elements which constitute the devices of the present invention may be placed in a single continuous flow path. Alternatively, the measurement methods of the present invention can be carried out by constructing each element as a discontinuous space and allowing a reaction solution to travel between the elements.

The devices of the present invention may be used in combination with an additional structural body for carrying out the measurement methods described above. Examples of an additional structural body that can be combined with the devices of the present invention are listed below.

Structural body for sorting samples
Structural body for diluting samples
Structural body for recording measurement results
Structural body for displaying measurement results

Structural body for printing measurement results

All prior art documents cited herein are incorporated by reference. The present invention is illustrated in detail below.

Examples

[Example 1] Acceleration of antigen-antibody reaction

(1) Preparation of an anti-AFP antibody-sensitized latex reagent

0.1 mg of an anti-$\alpha$-fetoprotein (AFP) antibody (Dako) was dissolved in 1 ml of glycine buffer (containing 50 mM glycine, 50 mM sodium chloride, and 0.09% sodium azide; hereinafter abbreviated as "GBS"), and 1 ml of 2.0-$\mu$m latex (Sekisui Chemical; 1% solid suspension) was added thereto. After the resulting mixture was stirred at 37°C for 2 hours, the sensitized latex was centrifuged and supernatant was discarded. The precipitate was suspended in 1 ml of glycine buffer containing 0.5% bovine serum albumin (0.5% BSA-GBS) to prepare an anti-AFP antibody-sensitized latex reagent.

(2) Measuring device

The affinity substance (AFP) was measured based on the antigen-antibody reaction using the device shown in Fig. 1 (A). The device shown in Fig. 1 is equipped with temperature control machinery 2 and dispensing/stirring vessel 1 which dispenses and mixes samples with reagent 1 (buffer) and then dispenses reagent 2 (latex reagent) and mixes to prepare reaction mixtures. However, when a single-reagent system is used, it is possible to omit dispensing reagent 1 (buffer). Then, the reaction mixtures are transferred to reaction vessel 3 (pulse-application vessel), and voltage pulses are applied to the reaction mixtures via electrodes 4 for several seconds to several tens of seconds. Carrier particles form pearl chains when placed in an electric field. After application of voltage pulses, the reaction mixtures are diluted in dilution vessel 5, and the state of carrier agglutination is measured using particle sizer 6. A cross-sectional view of the pulse-application vessel is shown in Fig. 1 (B). The distance between electrodes is 0.8 mm; electrode thickness is 0.03 mm; and electrode length is 20 mm.

(3) Sample measurement

An AFP antigen solution was diluted with 0.5% BSA-GBS to prepare sample solutions containing 0, 0.0075, and 0.015 ng/ml antigen. 3 $\mu$l of these samples and 3 $\mu$l of the anti-AFP antibody-sensitized latex reagent described above were transferred into test tubes. The mixtures were agitated. Immediately after 20 seconds of incubation at 45, 62, or 80°C, the mixtures were injected into the electrode-attached reaction vessel. Alternating voltage pulses (rectangular wave) with a frequency of 200 KHz were applied for 30 seconds using the device described in (2) to provide an electric field strength of $\pm$12 V/mm. Immediately after 30 seconds of application, the electric field was removed and the reaction solutions were diluted with physiological saline. The particle size distribution of latex particles was determined using Coulter Multisizer. The latex agglutination ratio (AR; %) was determined according to the following equation:

$$AR = \text{(number of particles that form agglutinates of two or more particles)} / \text{(total number of particles)} \times 100\ (\%)$$

(4) Control measurement

Control 1: 3 $\mu$l each of the respective samples shown in (3) and the anti-AFP antibody-sensitized latex reagent were transferred into test tubes. The resulting mixtures were incubated at 25°C for 20 seconds, and then injected into the electrode-attached reaction vessel. High frequency voltage was applied in the same way as described in (3). The procedure used was the same as described in (3) except that incubation was carried out at 25°C.. The result is shown as "Comparison example 1" in Fig. 2.

Control 2: 3 $\mu$l each of the respective samples shown in (3) and the anti-AFP antibody-sensitized latex reagent were transferred into test tubes. The resulting mixtures were incubated at 37°C for 20 minutes. 0.5 $\mu$l of the reaction solutions were diluted with 20 ml of physiological saline 20 ml. The particle size distribution of latex particles was measured using Coulter Multisizer in the same way as described in (3) to determine the agglutination ratio. The result is shown as "Comparison example 2" in Fig. 2.

(5) Results

The measurement results for each sample and control are shown in Fig. 2. It was found that the agglutination ratio was higher under the conditions of the present invention, where the reaction mixtures were incubated at high temperature (45, 62, or 80°C) for 20 seconds prior to voltage pulse application, as compared with the conventional method where the reaction mixtures were prepared at room temperature (at 25°C) prior to voltage pulse application and high temperature treatment was not used (control 1; "Comparison example 1" of Fig. 2). Agglutination was not detectable in a low concentration range of 0.015 pg/ml after 20 minutes of incubation at 37°C in control 2 ("Comparison example 2" of Fig. 2)) using a conventional latex agglutination method without applying voltage pulses. These results demonstrate that the method of the present invention enables higher sensitivity measurement than the conventional method (without temperature treatment prior to voltage pulse application).

[Example 2] Acceleration of antigen-antibody reaction

(1) Preparation of an anti-AFP antibody-sensitized latex reagent

An anti-AFP antibody-sensitized latex reagent was prepared in the same way as described in Example 1.

(2) Measuring device

The affinity substance (antigen-antibody reaction) was measured using the device shown in Fig. 1.

(3) Sample measurement

An AFP antigen solution was diluted with 0.5% BSA-GBS to prepare sample solutions containing 0, 0.0075, and 0.015 ng/ml antigen. 3 μl of these samples and 3 μl of the anti-AFP antibody-sensitized latex reagent described above were transferred into test tubes. The resulting mixtures were agitated. Immediately after 5, 20, or 180 seconds of incubation at 62°C, the mixtures were injected into the electrode-attached reaction vessel. Pearl chains were formed by applying an alternating voltage (rectangular wave) with a frequency of 200 KHz for 30 seconds using the device described above to provide an electric field strength of $\pm$12 V/mm. Immediately after 30 seconds of application, the electric field was removed and the reaction solution was diluted with physiological saline. The particle size distribution of latex particles was measured using Coulter Multisizer. The latex agglutination ratio (AR; %) was determined according to the following equation:

$$AR = \text{(number of particles that form agglutinates of two or more particles)} / \text{(total number of particles)} \times 100 \ (\%)$$

(4) Control measurement

Measurements were carried out in the same way as described in (3) except that high temperature treatment was not used (0 second) prior to voltage pulse application.

(5) Results

The results are shown in Fig 3. The agglutination ratio was higher under the conditions of the present invention shown as "5 sec", "20 sec", and "180 sec" (i.e., the reaction mixture is incubated at a high temperature (62°C) for 5, 20, or 180 seconds prior to voltage pulse application), as compared with when the conventional method (no high temperature treatment prior to voltage pulse application) shown as "0 sec" was used. Specifically, it was demonstrated that the present invention can achieve higher sensitivity in measurement than the conventional method.

[Example 3] Acceleration of antigen-antibody reaction

(1) Preparation of a PSA antibody-sensitized latex reagent (reagent 2)

0.1 mg of an anti-PSA antibody (Dako) was dissolved in 1 ml of glycine buffer (containing 50 mM glycine, 50 mM sodium chloride, and 0.09% sodium azide; hereinafter abbreviated as "GBS"), and 1 ml of 2.0-$\mu$m latex (Sekisui Chemical; 1% solid suspension) was added thereto. After the resulting mixture was stirred at 37°C for 2 hours, the sensitized latex was centrifuged and supernatant was discarded. The precipitate was suspended in 1 ml of glycine buffer containing 0.5% bovine serum albumin (0.5% BSA-GBS) to prepare an anti-PSA antibody-sensitized latex reagent.

(2) Preparation of Tris hydrochloride buffer containing PEG20000 (reagent 1)

A reaction-accelerating reagent was prepared, which was 50 mM Tris hydrochloride buffer (containing 50 mM Tris, 50 mM sodium chloride, and 0.09% sodium azide (pH 8.4)) containing 0.5% bovine serum albumin and 0.1 to 1.0% polyethylene glycol (molecular weight 20000; hereinafter abbreviated as PEG20000).

(3) Preparation of Tris hydrochloride buffer as a control

A reagent containing the same ingredients as described in (2) except that PEG20000 was prepared as a control.

(4) Measuring device

The affinity substance (antigen-antibody reaction) was measured using the device shown in Fig. 1. The temperature was set to room temperature on temperature control machinery 2.

(5) Sample and control measurements

A PSA antigen solution was diluted with 0.5% BSA-GBS to prepare sample solutions containing 0 and 9.5 ng/ml PSA. After 1 $\mu$l of these samples and 3 $\mu$l of Tris hydrochloride buffer containing 0.5% BSA and 0 to 1.0% PEG20000 were combined, 3 $\mu$l of the anti-PSA antibody-sensitized latex reagent described above was added into test tubes and agitated. The mixtures were immediately injected into the electrode-attached reaction vessel. Alternating voltage (rectangular wave) with a frequency of 200 KHz was applied for 30 seconds using the device described above to provide an electric field strength of $\pm$12 V/mm. Immediately after 30 seconds of application, the electric field was removed and the reaction solutions were diluted with physiological saline. The particle size distribution of latex particles was measured using Coulter Multisizer. The latex agglutination ratio (AR; %) was determined according to the following equation:

$$AR = \text{(number of particles that form agglutinates of two or more particles)} / \text{(total number of particles)} \times 100\ (\%)$$

(6) Results

The results are shown in Fig. 4. Fig. 4 shows that the agglutination ratio is increased when PEG, a water-soluble polymer, is used. This finding demonstrates that the present invention can achieve higher sensitivity in measurement as compared with the control method.

[Example 4] Acceleration of agglutination reaction

(1) Preparation of an anti-AFP antibody-sensitized latex reagent

An anti-AFP antibody-sensitized latex reagent was prepared in the same way as described in Example 1.

(2) Measuring device

AFP was measured based on the antigen-antibody reaction using the device shown in Fig. 5 (A). The device shown in Fig. 5 (A) is equipped with temperature control machinery and a dispensing-stirring vessel which dispenses and mixes samples with reagent (buffer), and then dispenses reagent 2 (latex reagent) and mixes to prepare reaction mixtures. However, when a single-reagent system is used, it is possible to omit buffer dispensing. Then, the reaction mixtures are transferred to reaction vessel 2 (pulse-application vessel), and voltage pulses are applied to the reaction mixtures *via* electrodes 3 for several seconds to several tens of seconds. During application of voltage pulses, the reaction vessel is cooled to 4°C by the temperature control unit. The reaction mixtures after voltage pulse application are diluted in dilution vessel 5, and the state of carrier agglutination is measured using particle sizer 6. Fig. 5 (B) is a diagram showing a cross-sectional view of the pulse-application vessel. The distance between electrodes is 0.8 mm; electrode thickness is 0.03 mm; and electrode length is 20 mm.

(3) Sample measurement

An AFP antigen solution was diluted with 0.5% BSA-GBS to prepare sample solutions containing 0 and 0.0075 ng/ml AFP. 3 $\mu$l of these samples and 3 $\mu$l of the anti-AFP antibody-sensitized latex reagent described above were transferred into test tubes. The resulting mixtures were agitated, and immediately injected into the electrode-attached reaction vessel. Alternating voltage (rectangular wave) with a frequency of 200 KHz was applied to the reaction solutions for 30 seconds using the device described above to provide an electric field strength of $\pm$12 V/mm. During application of voltage pluses, the reaction vessel was kept at 4°C. Immediately after 30 seconds of application, the electric field was removed and the reaction solutions were diluted with physiological saline. The particle size distribution of latex particles was measured using Coulter Multisizer. The latex agglutination ratio (AR; %) was determined according to the following equation:

$$AR = (\text{number of particles that form agglutinates of two or more particles}) / (\text{total number of particles}) \times 100\ (\%)$$

Measurement was achieved by repeating the same manipulation as described above five times. Mean and mean $\pm$ 2.6SD were determined from the measurement results and are shown in Fig. 6.

(4) Control measurement

Control 1: The respective samples shown in (3) and the anti-AFP antibody-sensitized latex reagent were treated by the same procedure described in (3) except that the temperature of the reaction vessel was 22°C. The result is shown in Fig. 7.

Control 2: 3 $\mu$l of the respective samples shown in (3) and the anti-AFP antibody-sensitized latex reagent were transferred into test tubes. The resulting mixtures were incubated at 37°C for 20 minutes. 0.5 $\mu$l of the reaction solutions were diluted with 20 ml of physiological saline. The particle size distribution of latex particles was measured using Coulter Multisizer in the same way as described in (3) to determine the agglutination ratio. Measurement was achieved by repeating the same manipulation described above in (3) 5 times. The result is shown in Fig. 8.

(5) Results

When a value obtained by measuring a certain concentration of antigen is distinguished from a value measured in the absence of antigen (background signal), the antigen can be measured at this concentration. The minimal value of measurable antigen concentration is the detection limit. For example, when the range of value A does not overlap the range of value B, 0.0075 ng/ml or higher concentration of antigen can be detected.
Measured value A: average value +2.6SD of agglutination ratio determined when the antigen concentration was 0 ng/ml, which was obtained by repeating the measurement using 0 ng/ml and 0.0075 ng/ml antigen.
Measured value B: average value -2.6SD of agglutination ratio determined when the antigen concentration was 0.0075 ng/ml.
The detection limits under the respective conditions were compared using the results shown in Figs. 6, 7, and 8. The detection limit for the method of the present invention (Fig. 6) was found to be 0.0075 ng/ml. Meanwhile, it was found that, this concentration was no detectable by the conventional methods (Figs. 7 and 8) as controls. This result demonstrates that the present invention that comprises keeping the reaction solution cool during application of voltage pulses enables rapid, higher-sensitivity measurement as compared with the conventional methods.

[Example 5] Acceleration of agglutination reaction

(1) Preparation of an anti-PSA antibody-sensitized latex reagent (reagent 2)

An anti-PSA antibody-sensitized latex reagent was prepared in the same way as described in Example 3.

(2) Preparation of Tris hydrochloride buffer (reagent 1)

Reagent 1 which comprises a Tris hydrochloride buffer (containing 50 mM Tris, 50 mM sodium chloride, 0.09% sodium azide, and 0.25% PEG20000, (pH 8.4)) containing 0.5, 2.5, 5, 7.5, or 10% bovine serum albumin (hereinafter abbreviated as BSA) was prepared.

(3) Measuring device

The affinity substance (antigen-antibody reaction) was measured using the device shown in Fig. 1. The temperature was set to room temperature on temperature control machinery 2.

(4) Measurement

A PSA antigen solution was diluted with 0.5% BSA-GBS to prepare sample solutions containing 0, 9.5, or 32 ng/ml PSA. 1 $\mu$l of these samples and 3 $\mu$l of a Tris buffer containing 0.5, 2.5, 5, 7.5, or 10% BSA were combined, and the resulting mixtures were agitated. Then, 3 $\mu$l of the anti-PSA antibody-sensitized latex reagent described above was added to the mixtures. Immediately after mixing, the mixtures were injected into the electrode-attached reaction vessel. Alternating voltage (rectangular wave) with a frequency of 200 KHz was applied for 30 seconds using the device described above to provide an electric field strength of $\pm$ 12 V/mm. Immediately after application, the electric field was removed, and the reaction solution was diluted with physiological saline. The particle size distribution of latex particles was measured using Coulter Multisizer. The latex agglutination ratio (AR; %) was determined according to the following equation:

$$AR = (\text{number of particles that form agglutinates of two or more particles}) / (\text{total number of particles}) \times 100\ (\%)$$

BSA concentrations in the final reaction solutions were 0.5, 1.4, 2.4, 3.5, and 4.6%. The final reaction solution containing 0.5% BSA was used as a control.

(5) Measurement of the temperature of reaction solution

The temperature change in the reaction solution in which pearl chain formation occurred was monitored by the measurement method described above.

(6) Measurement of the viscosity of the final reaction solution

The viscosity of the final reaction solutions (0.5 to 4.6% BSA) described above and the reaction solutions containing 0.3 and 6.8% BSA was measured at 4 to 52°C using an oscillating viscometer (VISCOMATE).

(7) Results

[Table 1]

| BSA concentration in the final reaction solution | Before application | Immediately before removal of electric field |
|---|---|---|
| 0.5% | 24°C | 37°C |
| 1.4% | 24°C | 38°C |
| 2.4% | 25°C | 37°C |
| 3.5% | 25°C | 38°C |

(continued)

| BSA concentration in the final reaction solution | Before application | Immediately before removal of electric field |
|---|---|---|
| 4.6% | 24°C | 37°C |

The results are shown in Figs. 9 and 10, and Table 1. As seen in Fig. 9, the agglutination ratio at each concentration increased as the BSA concentration was increased from 0.5 to 2.4% in the final reaction solution. The agglutination ratio had a tendency to increase even in the absence of PSA (0 ng/ml (blank)). Therefore, the agglutination ratios were compared after normalization using the blank. It was thus demonstrated that the agglutination ratio markedly increased when the BSA concentration was increased from 0.5% to 2.4% in the final reaction solution. Specifically, it was demonstrated that higher-sensitivity measurement could be achieved by the present invention.

Fig. 10 shows the relationship between BSA concentration in the final reaction solution and viscosity of the solution, and the relationship between temperature and viscosity of the reaction solution. First, Figs. 9 and 10 show that the agglutination ratio increases as the BSA concentration increases from 0.5% to 2.4% in the final reaction solution when the temperature is not controlled during pearl chain formation. The viscosity of the final reaction solution increased from 0.75 to 0.9 mPas along with the rise in BSA concentration. Specifically, it was demonstrated that high sensitivity measurement could be achieved by adjusting the viscosity of the final reaction solution to be within the range of 0.75 to 0.9 mPas.

Table 1 shows results of measuring the temperature change of the reaction solution during pearl chain formation upon application of alternating voltage. Before application, the temperature of the reaction solution was room temperature (about 25°C). The temperature increased to about 37°C after application. Meanwhile, when a conventional method was used, the BSA concentration in the final reaction solution was about 0.5%. However, the viscosity of the reaction solution was less than 0.8 mPas (0.6 to 0.75 mPas) because of the increase in temperature resulted from application of voltage pulses. Thus, it was demonstrated that higher sensitivity measurement could be achieved by applying alternating voltage under conditions where the viscosity of the reaction solution was adjusted to be within the range of 0.8 to 0.9 mPas.

Furthermore, Table 1 shows that the viscosity of the reaction solution of the present invention described in Example 4, where the reaction solution was kept cool during application of voltage pulses, was 1.4 mPas. These findings show that higher sensitivity measurement can be achieved by applying alternating voltage under conditions where the viscosity of the reaction solution is adjusted to be within the range of 0.8 to 3 mPas. The results described above demonstrate that the viscosity is preferably in the range of 1 to 3 mPas, more preferably in the range of 1 to 2 mPas to improve sensitivity.

[Example 6]

(1) Preparation of an anti-AFP antibody-sensitized latex reagent

0.1 mg of an anti-AFP antibody (Dako) was dissolved in 1 ml of glycine buffer (containing 50 mM glycine, 50 mM sodium chloride, and 0.09% sodium azide; hereinafter abbreviated as GBS), and 2.06-μm latex (Polyscience; 1.0% solid suspension) was added thereto. After the resulting mixture was stirred at 37°C for 2 hours, the sensitized latex was centrifuged and supernatant was discarded. The precipitate was suspended in 1 ml of glycine buffer containing 0.5% bovine serum albumin (0.5% BSA-GBS) to prepare an anti-AFP antibody-sensitized latex reagent.

(2) Measuring device

A specific biological agglutination reaction (antigen-antibody reaction) was measured using the device shown in Fig. 11 (A). The device shown in Fig. 11(A) is equipped with a dispensing/stirring vessel with temperature control machinery 1, which dispenses and mixes samples with reagent 1 (buffer: to be used in the dual reagent system; not required in the single reagent system/latex reagent alone), and then dispenses reagent 2 (latex reagent) and mixes. The reaction solution was mixed in the dispensing/stirring vessel with temperature control machinery 1, and then transferred into reaction vessel 3 (pulse-application vessel). Voltage pulses were applied to the solution *via* electrodes 4 for several seconds to several tens of seconds to form pearl chains. After pearl chain formation, the reaction solution was diluted (dilution vessel 5) and the state of carrier agglutination was measured using particle sizer 6. (Temperature control machinery 1 and 2 were not used.)

The outline of dilution vessel 5 is shown in Fig. 11 (B). A diluent was dispensed in dilution vessel 103, while the reaction solution is dispensed between paired electrodes 101.

(3) Sample measurement

Measurement was carried out using sample solutions of AFP control sera L (15.6 ng/ml), M (125 ng/ml), and H (1000 ng/ml), and a serum-free sample solution (0 ng/ml). 3 $\mu$l of the samples and 3 $\mu$l of the anti-AFP antibody-sensitized latex reagent described above were transferred into test tubes. The resulting mixtures were agitated, and immediately injected into the electrode-attached reaction vessel. Pearl chains were formed by applying an alternating voltage (rectangular wave) with a frequency of 200 KHz for 30 seconds using the device described above to provide an electric field strength of $\pm$12 V/mm. Immediately after 30 seconds of application, the electric field was removed and the reaction solution was diluted with physiological saline. The reaction solution was diluted by adding the solution to a diluent while a $\pm$ 0.7 V alternating voltage (rectangular wave) with a frequency of 200 KHz was applied between the electrodes in the diluent using the device shown in Fig. 1 (B). Then, the particle size distribution of latex particles was measured using Coulter Multisizer. The latex agglutination ratio (AR) was determined according to the following equation:

$$AR = (\text{number of particles that form agglutinates of two or more particles}) / (\text{total number of particles}) \times 100 \ (\%)$$

(4) Control measurement

The respective samples shown in (2) and the anti-AFP antibody-sensitized latex reagent were used. Except that the reaction solution was diluted without an electric field, the same procedure described in (3) was used in the step of diluting the reaction solution after pearl chain formation. The result is shown as the "control method" in Fig. 12.

(5) Results

The results are shown in Fig. 12. According to the results of measuring 1000 ng/ml AFP, the agglutination ratio was determined to be 53.4 and 40.9% by the method of the present invention and by the conventional method as a control, respectively. Specifically, as compared with the conventional method, the method of the present invention could reduce about 20% of the disruption of agglutinates due to dilution of the reaction solution, namely the disruption of specific agglutinates of carrier particles. According to the results of measuring 0 ng/ml AFP, the agglutination ratio was determined to be 7.4% and 10.6% by the method of the present invention and by the conventional method as a control, respectively. The agglutination ratio determined for the 0 ng/ml AFP sample can be regarded as the background level due to nonspecific agglutination. Specifically, nonspecific agglutination is suppressed by the method of the present invention. The results described above show that the measurement method of the present invention gives a great and linear gradient of the agglutination ratio. Therefore, the present invention enables high-sensitivity measurement over a broader concentration range.

[Example 7]

(1) Preparation of an anti-AFP antibody-sensitized latex reagent

An anti-AFP antibody-sensitized latex reagent was prepared in the same way as described in Example 6.

(2) Measuring device

The affinity substance (antigen-antibody reaction) was measured using the device shown in Fig. 11.

(3) Sample measurement

A sample solution of AFP control serum L (15.6 ng/ml) and a serum-free sample solution (0 ng/ml) were measured. 3 $\mu$l of the samples and 3 $\mu$l of the anti-AFP antibody-sensitized latex reagent described above were transferred into test tubes. The resulting mixtures were agitated, and immediately injected into the electrode-attached reaction vessel. Pearl chains were formed by applying an alternating voltage (rectangular wave) with a frequency of 200 KHz for 30 seconds using the device described above to provide an electric field strength of $\pm$ 12 V/mm. Immediately after 30 seconds of application, the electric field was removed and the reaction solution was diluted with 20 ml of physiological saline. The reaction solution was diluted by adding the solution to a diluent while a $\pm$ 0.7 V alternating voltage (rectangular wave) with a frequency of 200 KHz was applied between the electrodes in the diluent using the device shown in Fig. 11 (B). The particle size distribution of latex particles was measured using Coulter Multisizer. The latex agglutination ratio (AR) was determined according to the following equation:

$$AR = \text{(number of particles that form agglutinates of two or more particles)} / \text{(total number of particles)} \times 100 \, (\%)$$

Measurement was achieved by repeating the above-described manipulation 5 times. Mean and mean $\pm$ 2SD were determined from the measurement results.

(4) Control measurement

Control 1: The respective samples shown in (3) and the anti-AFP antibody-sensitized latex reagent were used. Except that the reaction solution was diluted without an electric field, the same procedure described in (3) was used in the step of diluting the reaction solution after pearl chain formation. The result is shown as "control method 1" in Fig. 13.
Control 2: The respective samples shown in (3) and the anti-AFP antibody-sensitized latex reagent were used. The same procedure described in (3) was used except that the diluent used is a diluent to which an electric field has been applied under the same conditions described in (3) for the diluent of the reaction solution. The result is shown as "control method 2" in Fig. 13.

(5) Results

The results are shown in Fig. 13. According to the results of measuring 15.6 ng/ml AFP, the agglutination ratio was determined to be 35.7% and 32.6% by the method of the present invention and by the conventional method as a control, respectively. Specifically, as compared with the conventional method, the method of the present invention could reduce about 10% of the disruption of agglutinates due to dilution of the reaction solution, namely the disruption of specific agglutinates of carrier particles. According to the measurement result for 0 ng/ml AFP, the agglutination ratio was determined to be 5.6% and 11.1 % by the method of the present invention and by the conventional method, respectively. The agglutination ratio determined for the 0 ng/ml AFP sample can be regarded as the background level due to nonspecific agglutination. Specifically, nonspecific agglutination is suppressed by the method of the present invention. Furthermore, according to the result of the measurement for AFP 15.6 ng/ml in quintuplicate, the CV value was determined to be 1.16% and 4.28% by the method of the present invention and by the conventional method, respectively. This shows that the reproducibility of measurement was improved significantly. Thus, it is demonstrated that the present invention enables high-sensitivity, high-accuracy measurement.

[Example 8] Acceleration of antigen-antibody reaction

(1) Preparation of an anti-PSA antibody-sensitized latex reagent (reagent 2)

0.1 mg of an anti-PSA antibody (Dako) was dissolved in 1 ml of glycine buffer (containing 50 mM glycine, 50 mM sodium chloride, and 0.09% sodium azide; hereinafter abbreviated as GBS), and 2.06-$\mu$m latex (Polyscience; 1% solid suspension) was added thereto. After the resulting mixture was stirred at 37°C for 2 hours, the sensitized latex was centrifuged and supernatant was discarded. The precipitate was suspended in 1 ml of glycine buffer containing 0.5% bovine serum albumin (0.5% BSA-GBS) to prepare an anti-PSA antibody-sensitized latex reagent.

(2) Preparation of Tris hydrochloride buffer (reagent 1)

A reaction-accelerating reagent was prepared: 50 mM Tris hydrochloride buffer (containing 50 mM Tris, 50 mM sodium chloride, and 0.09% sodium azide (pH 8.4)) containing 0.5% bovine serum albumin and 0.25% polyethylene glycol (molecular weight 20000; hereinafter abbreviated as PEG20000).

(3) Measuring device

The affinity substance (antigen-antibody reaction) was measured using the device shown in Fig. 11.

(4) Sample measurement

Sample solutions of PSA control serum L (9.5 ng/ml) and M (32 ng/ml), and a serum-free sample solution (0 ng/ml) were measured. 1 µl of the samples and 3 µl each of the Tris hydrochloride buffer and anti-PSA antibody-sensitized latex reagent described above were transferred into test tubes. The resulting mixtures were agitated, and immediately injected into the electrode-attached reaction vessel. Pearl chains were formed by applying an alternating voltage (rectangular wave) with a frequency of 200 KHz for 30 seconds using the device described above to provide an electric field strength of ± 12 V/mm. Immediately after 30 seconds of application, the electric field was removed and the reaction solution was diluted with 20 ml of GBS. The dilution fold was about 3300. The reaction solution was diluted by adding the solution to a diluent while a ± 0.7 V alternating voltage (rectangular wave) with a frequency of 200 KHz was applied between the electrodes in the diluent using the device shown in Fig. 11 (B). The particle size distribution of latex particles was measured using Coulter Multisizer. The latex agglutination ratio (AR) was determined according to the following equation:

$$AR = \text{(number of particles that form agglutinates of two or more particles) / (total number of particles) x 100 (\%)}$$

(5) Control measurement

Control 1: The respective samples shown in (4), Tris buffer, and the anti-PSA sensitized latex reagent were used. Except that the reaction solution was diluted without an electric field, the same procedure described in (4) was used in the step of diluting the reaction solution after pearl chain formation. The result is shown as "control method 1" in Fig. 14.
Control 2: The respective samples shown in (4), Tris buffer, the and anti-PSA sensitized latex reagent were used. The same procedure described in (4) was used except that the diluent used is a diluent to which an electric field has been applied under the same conditions described in (4) for the diluent of the reaction solution. The result is shown as "control method 2" in Fig. 14.

(6) Results

The results are shown in Fig. 14. According to the measurement result for 32 ng/ml PSA, the agglutination ratio was determined to be 35.7% and 30.3% by the method of the present invention and by the conventional method as a control, respectively. Specifically, as compared with the conventional method, the method of the present invention could reduce about 20% of the disruption of agglutinates due to dilution of the reaction solution, namely the disruption of specific agglutinates of carrier particles. Furthermore, according to the result of measuring 0 ng/ml PSA, the agglutination ratio was determined to be 1.73% and 2.45% by the method of the present invention and by the conventional method, respectively. The agglutination ratio determined for the 0 ng/ml PSA sample can be regarded as the background level due to nonspecific agglutination. Specifically, nonspecific agglutination is suppressed by the method of the present invention. The results described above show that the measurement method of the present invention gives a great and linear gradient of the agglutination ratio. Thus, it was demonstrated that the present invention enabled high-sensitivity measurement over a broader concentration range.

[Example 9]

(1) Preparation of an anti-AFP antibody-sensitized latex reagent

An anti-AFP antibody-sensitized latex reagent was prepared in the same way as described in Example 6. Three types of anti-AFP antibody-sensitized latex reagents were prepared using suspensions (1.0% solid) of 2.0-µm (Sekisui Chemical), 3-µm (Polyscience) and 4.5-µm (Polyscience) latexes.

(2) Measuring device

The affinity substance (antigen-antibody reaction) was measured using the device shown in Fig. 11.

(3) Sample measurement

Measurement was carried out using the three types of latex reagents described above by the same procedure described in Example 6.

(4) Control measurement

The respective samples shown in (3) and the anti-AFP antibody-sensitized latex reagent were used. Except that the reaction solution was diluted without an electric field, the same procedure described in (3) was used in the step of diluting the reaction solution after pearl chain formation. The result is shown as the "control method" in Figs. 15, 16, and 17.

(5) Results

The results obtained using 2.0-, 3-, and 4.5-$\mu$m latex reagents are shown in Figs. 15, 16, and 17, respectively.

According to the measurement result for 15.6 ng/ml AFP obtained using the 2.0-$\mu$m latex reagent, the agglutination ratio was determined to be 35.8% and 24.1% by the method of the present invention and by the conventional method as a control, respectively. Thus, the method of the present invention could reduce 30% of the disruption of specific agglutinates (Fig. 15). As seen in Fig. 15, according to the measurement result for 0 ng/ml AFP, the agglutination ratio was determined to be 5.3% and 8.0% by the method of the present invention and by the conventional method, respectively.

According to the measurement result for 15.6 ng/ml AFP obtained using the 3-$\mu$m latex reagent, the agglutination ratio was determined to be 29.5% and 23.6% by the method of the present invention and by the conventional method as a control, respectively. Thus, the method of the present invention could reduce 20% of the disruption of specific agglutinates (Fig. 16). As seen in Fig. 16, according to the measurement result for 0 ng/ml AFP, the agglutination ratio was determined to be 13.6% and 17.8% by the method of the present invention and by the conventional method, respectively.

Furthermore, according to the measurement result for 15.6 ng/ml AFP obtained using the 4.5-$\mu$m latex reagent, the agglutination ratio was determined to be 11.5% and 3.0% by the method of the present invention and by the conventional method as a control, respectively. Thus, the method of the present invention could reduce 70% of the disruption of specific agglutinates (Fig. 17). As seen in Fig. 17, according to the measurement result for 0 ng/ml AFP, the agglutination ratio was determined to be 4.1% and 2.5% by the method of the present invention and by the conventional method, respectively.

Specifically, it was found that, as compared with the conventional method, the present invention could reduce the disruption of agglutinates of carrier particles. Furthermore, nonspecific agglutination caused by dilution could be suppressed in the present invention. Thus, it was demonstrated that the present invention enables high sensitivity measurement.

[Example 10]

(1) Preparation of an anti-AFP antibody-sensitized latex reagent

An anti-AFP antibody-sensitized latex reagent was prepared by chemically linking an anti-AFP antibody with latex particles using 0.1 ml (2.2 mg) of the anti-AFP antibody (Dako), 0.5 ml of 1.716-$\mu$m latex (Polyscience; 2.5% solid suspension), and a carbodiimide kit (Polyscience) according to the manual attached to the kit.

(2) Measuring device

The affinity substance (antigen-antibody reaction) was measured using the device shown in Fig. 1.

(3) Sample measurement

(3) Sample measurement

A sample solution of AFP control serum H (1000 ng/ml) was measured. 3 $\mu$l of the sample and 3 $\mu$l of the above-described anti-AFP antibody-sensitized latex reagent were transferred into test tubes. The resulting mixture was agitated, and immediately injected into the electrode-attached reaction vessel. Pearl chains were formed by applying an alternating voltage (rectangular wave) with a frequency of 200 KHz for 30 seconds using the device described above to provide an electric field strength of±12 V/mm. Immediately after 30 seconds of application, the electric field was removed and 16 µl of a 0.25 to 25% glutaraldehyde solution (hereinafter abbreviated as GA solution) was added to the reaction solution. The resulting mixture was incubated at 37°C for 60 minutes, and then diluted with 20 ml of physiological saline. The particle size distribution of latex particles in the diluted reaction solution was measured using Coulter Multisizer. The latex agglutination ratio (AR) was determined according to the following equation:

$$AR = \text{(number of particles that form agglutinates of two or more particles)} / \text{(total number of particles)} \times 100\ (\%)$$

Next, the remaining diluted reaction solution was sonicated for 30 or 60 seconds, and then the particle size distribution was determined by the same procedure (severe disruption test).

(4) Control measurement

The respective samples shown in (3) and the anti-AFP antibody-sensitized latex reagent were used. The same procedure described in (3) was used, except that the samples were diluted with GA-minus physiological saline. The result is shown as "0% GA" in Fig. 18.

(5) Results

The results are shown in Fig. 18. Agglutination ratios immediately after dilution are compared with each other. The agglutination ratio was 21 % when the reaction solution was diluted after GA treatment. The ratio was 16% without GA treatment. Thus, 5% disruption was confirmed. Furthermore, the severe disruption test revealed that the disruption was markedly improved with 25% GA treatment. The finding described above demonstrates that, when compared with conventional methods, the present invention can reduce the disruption of specific agglutinates of carrier particles and thus enables high-sensitivity measurement.

[Example 11]

(1) Preparation of an anti-AFP antibody-sensitized latex reagent

An anti-AFP antibody-sensitized latex reagent was prepared by the same procedure described in Example 10.

(2) Measuring device

The affinity substance (antigen-antibody reaction) was measured using the device shown in Fig. 11.

(3) Sample measurement

A sample solution of AFP control serum H (1000 ng/ml) was measured. 3 µl of the sample and 3 µl of the above-described anti-AFP antibody-sensitized latex reagent were transferred into a test tube. The resulting mixture was agitated, and immediately injected into the electrode-attached reaction vessel. Pearl chains were formed by applying an alternating voltage (rectangular wave) with a frequency of 200 KHz for 30 seconds using the device described above to provide an electric field strength of±12 V/mm. Immediately after 30 seconds of application, the electric field was removed and 16 µl of a 25% glutaraldehyde solution (hereinafter abbreviated as GA solution) was added to the reaction solution. The resulting mixture was incubated at 37°C for 0 to 60 seconds and diluted with physiological saline. The particle size distribution of latex particles in the diluted reaction solution was measured using Coulter Multisizer. The latex agglutination ratio (AR) was determined according to the following equation:

$$AR = \text{(number of particles that form agglutinates of two or more particles)} / \text{(total number of particles)} \times 100\ (\%)$$

Next, the remaining diluted reaction was sonicated for 30 or 60 seconds, and then the particle size distribution was determined by the same procedure (severe disruption test).

(4) Control measurement

The respective samples shown in (3) and the anti-AFP antibody-sensitized latex reagent were used. The same procedure described in (3) was used, except that the solution was diluted with GA-minus physiological saline. The result is shown as "without GA" in Fig. 19.

(5) Results

The results are shown in Fig. 19. Agglutination ratios immediately after dilution are compared with each other. The agglutination ratio was 21 % regardless of the period of treatment (15, 30, and 60 seconds) when the reaction solution was diluted after 25% GA treatment. The ratio was 18% when the period of treatment is 0 sec (dilution immediately after mixing), while the ratio was 16% without GA treatment. Furthermore, the severe disruption test also revealed that disruption was comparably improved by 25% GA treatment (treatment period: 15 to 60 seconds). The finding described above demonstrates that, when compared with the conventional method, the present invention can reduce the disruption of specific agglutinates of carrier particles and thus enables high-sensitivity measurement.

[Example 12]

(1) Preparation of an anti-AFP antibody-sensitized latex reagent

An anti-AFP antibody-sensitized latex reagent was prepared by the same procedure described in Example 6. Three types of anti-AFP antibody-sensitized latex reagents were prepared using 1.0% solid suspensions of 2.0-$\mu$m (Sekisui Chemical), 2.8-$\mu$m (Polyscience), and 1.7-$\mu$m (Polyscience) latexes.

(2) Measuring device

The affinity substance (antigen-antibody reaction) was measured using the device shown in Fig. 11.

(3) Sample measurement

Measurement was carried out using the three types of latex reagents described above by the same procedure described in Example 11.

(4) Control measurement

The respective samples shown in (3) and the anti-AFP antibody-sensitized latex reagent were used. The same procedure described in (3) was used, except that the solution was diluted with GA-minus physiological saline. The result is shown as "control method" in Figs. 20, 21, and 22.

(5) Results

The results obtained using 2.0-$\mu$m, 2.8-$\mu$m, and 1.7-$\mu$m latex reagents are shown in Figs. 20, 21, and 22, respectively. The results shown in Figs. 20, 21, and 22 demonstrate that, when compared with the conventional method (diluent containing no binding enhancer), regardless of the particle diameter of latex, the present invention can reduce the disruption of specific agglutinates of carrier particles and thus enables high-sensitivity measurement.

[Example 13] Repeatability test

(1) Preparation of an anti-AFP antibody-sensitized latex reagent

An anti-AFP antibody-sensitized latex reagent was prepared by the same procedure described in Example 10.

(2) Measuring device

The affinity substance (antigen-antibody reaction) was measured using the device shown in Fig. 11.

(3) Measurement method

Sample solutions of AFP control sera L and M were measured. 3 µl of the samples and 3 µl of the above-described anti-AFP antibody-sensitized latex reagent were transferred into test tubes. The resulting mixtures were agitated, and immediately injected into the electrode-attached reaction vessel. Pearl chains were formed by applying an alternating voltage (rectangular wave) with a frequency of 200 KHz for 30 seconds using the device described above to provide an electric field strength of ± 12 V/mm. Immediately after 30 seconds of application, the electric field was removed and 16 µl of a glutaraldehyde solution (hereinafter abbreviated as GA solution) was added to the reaction solutions. The resulting mixtures were incubated at 37°C for 0 to 60 seconds and diluted with physiological saline. The particle size distribution of latex particles in the diluted reaction solutions was measured using Coulter Multisizer. The latex agglutination ratio (AR) was determined according to the following equation:

$$AR = (\text{number of particles that form agglutinates of two or more particles}) / (\text{total number of particles}) \times 100\ (\%)$$

Reproducibility test was achieved by repeating the above-described manipulation 10 times.

(4) Control measurement

The respective samples described in (3) and the anti-AFP antibody-sensitized latex reagent were used. The same procedure described in (3) was used, except that the solution was diluted with GA-minus physiological saline. The result is shown as "control method" in Table 2.

(5) Results

The results are shown in Table 2. When the present invention was used, the CV value for repeatability was about 2%. Meanwhile, when the control method was used, the CV value was in the range of 7 to 11% and thus showed great variations. The finding described above demonstrates that, when compared with the control method, the present invention enables high reproducibility measurement.

[Table 2]

| N=10 | Agglutination ratio (%) | | | |
| --- | --- | --- | --- | --- |
| | Control serum L | | Control serum M | |
| | Method of this invention | Control method | Method of this invention | Control method |
| 1 | 8.54 | 6.43 | 23.22 | 8.88 |
| 2 | 8.38 | 4.82 | 23.36 | 10.56 |
| 3 | 8.42 | 5.03 | 23.53 | 11.14 |
| 4 | 8.71 | 4.78 | 24.26 | 9.69 |
| 5 | 8.67 | 5.35 | 23.17 | 10.37 |
| 6 | 8.70 | 6.17 | 22.40 | 9.79 |
| 7 | 8.44 | 5.53 | 22.23 | 9.68 |
| 8 | 8.46 | 5.20 | 23.68 | 9.38 |
| 9 | 8.86 | 5.07 | 23.30 | 9.35 |
| 10 | 8.42 | 5.92 | 23.56 | 10.15 |
| Ave | 8.560 | 5.429 | 23.270 | 9.897 |
| SD | 0.165 | 0.571 | 0.593 | 0.665 |
| CV | **1.93%** | **10.52%** | **2.55%** | **6.72%** |

Industrial Applicability

The measurement methods and measuring devices of the present invention are useful for measuring various affinity substances. Specifically, the analysis of collected biological samples can provide useful information for diagnosing various diseases. More specifically, hormones, tumor markers, enzymes, drugs, and infectious pathogens, and antibodies thereto are routinely measured in medical institutions. All these measurement targets are included in the affinity substance of the present invention. Alternatively, it is possible to measure or detect microorganisms, drugs, and such in biological samples, food, or environmental samples according to the present invention.

**Claims**

1. A method for measuring an affinity substance, which comprises the steps of:

    (1) incubating a mixed reaction solution comprising the affinity substance to be measured and carrier particles that are bound to a binding partner having the activity to bind to the affinity substance to be measured;
    (2) applying voltage pulses to the reaction solution of step (1);
    (3) counting, after step (2), agglutinates of carrier particles formed through the binding with the affinity substance to be measured, or unagglutinated carrier particles that do not bind to the affinity substance to be measured, or both; and
    (4) determining, after step (3), the level of the substance to be measured based on either or both of the level of agglutinate formation and the level of unagglutinated carrier particles.

2. The method of claim 1, wherein the reaction solution is incubated at 37 to 90°C in step (1).

3. The method of claim 2, wherein the reaction solution is incubated at 40 to 90°C in step (1).

4. The method of claim 1, wherein the reaction solution contains a water-soluble polymer.

5. The method of claim 1, wherein the viscosity of the reaction solution is adjusted to 0.8 to 3 mPas in step (2).

6. The method for measuring an affinity substance according to claim 1, wherein step (2) is carried out at 0 to 20°C.

7. The method for measuring an affinity substance according to claim 6, wherein step (2) is carried out at 0 to 10°C.

8. The method of claim 1, wherein either or both of the agglutinates and unagglutinated carrier particles are counted using the three-dimensional information thereof as an indicator.

9. The method of claim 1, wherein the binding between the affinity substance and the binding partner is an antigen-antibody reaction.

10. The method of claim 9, wherein the affinity substance is an antigen and the binding partner is an antibody or a fragment comprising an antigen-binding domain of the antibody.

11. The method of claim 9, wherein the affinity substance is an antibody or a fragment comprising an antigen-binding domain of the antibody, and the binding partner is an antigen or a fragment comprising an epitope of the antigen.

12. The method of claim 1, wherein the voltage pulse is an alternating voltage pulse.

13. The method for measuring an affinity substance, which comprises the steps of:

    (1') incubating a reaction solution comprising an affinity substance to be measured and carrier particles that are bound to a binding partner having the activity to bind to at least the affinity substance to be measured before or after mixing with an agglutination reagent, wherein the
    carrier particles agglutinate via the agglutination reagent and the agglutination is inhibited by the affinity substance to be measured;
    (2') applying voltage pulses to the reaction solution of step (1') in the presence of the agglutination reagent;
    (3') counting, after step (2'), agglutinates of carrier particles formed through the binding with the agglutination reagent, or unagglutinated carrier particles whose agglutination is inhibited by the binding of the affinity substance to be measured, or both; and
    (4') determining, after step (3'), the level of the substance to be measured based on either or both of the level of agglutinate formation and the level of unagglutinated carrier particles.

14. The method of claim 13, wherein, after incubation of the reaction solution in step (1'), the agglutination reagent is mixed before step (2').

15. The method of claim 13, which comprises, after mixing the agglutination reagent, another incubation step before step (2').

16. The method of claim 13, wherein step (2') is carried out after the reaction solution is incubated in the presence of the agglutination reagent in step (1').

17. A device for agglutinating carrier particles, which comprises in a device a means for applying voltage pulses to a reaction solution comprising a particular substance and carrier particles that are bound to a binding partner having the activity to bind to the particular substance, a means of heating the reaction solution to a temperature within the range of 37 to 90°C.

18. A method for agglutinating carrier particles, which comprises in a method of applying voltage pulses to a reaction solution comprising a particular substance and carrier particles that bind to a binding partner having the activity to bind to the particular substance, keeping the temperature of the reaction solution within the range of 0 to 20°C during voltage application.

19. The method of claim 18, wherein the binding between the binding partner and the particular substance is an antigen-antibody reaction.

20. The method of claim 18, wherein the voltage pulse is an alternating voltage pulse.

21. The method of claim 18, wherein the water-soluble polymer is added to the reaction solution.

22. The method of claim 18, wherein the viscosity of the reaction solution is adjusted to 0.8 to 3 mPas.

23. The method of claim 18, which comprises the strep of incubating the carrier particles and the particular substance at 37 to 90°C before voltage pulse application.

24. A device for agglutinating carrier particles, which comprises in a device a means for applying voltage pulses to a reaction solution comprising a particular substance and carrier particles that bind to a binding partner having the activity to bind to the particular substance, a means of keeping the temperature of the reaction solution within the range of 0 to 20°C during voltage application.

25. A device for measuring the binding between an affinity substance and carrier particles that bind to a binding partner having the activity to bind to the affinity substance to be measured using as an indicator the agglutination of the carrier particles by the affinity substance or an agglutination reagent, comprising the elements of:

a: a space for retaining a reaction solution;
b: a means for incubating the reaction solution at 37 to 90°C;
c: a means for applying voltage pulses to the reaction solution;
d: a means for keeping the temperature of the reaction solution within the range of 0 to 20°C during voltage pulse application; and
e: a means for counting either or both of carrier particles and agglutinates of carrier particles in the reaction solution.

26. A method for diluting the reaction solution using a means of enhancing the binding between an affinity substance and a binding partner or the binding between an agglutination reagent and an binding partner before step (2) or (2') in a method of measuring an affinity substance, which comprises the steps of:

(1) applying voltage pulses to a mixed reaction solution comprising the affinity substance to be measured and carrier particles that bind to a binding partner having the activity to bind to the affinity substance to be measured;
(2) counting, after step (1), agglutinates of carrier particles formed through the binding with the affinity substance to be measured, or unagglutinated carrier particles that have not bound to the affinity substance to be measured, or both; and

(3) determining, after step (2), the level of the substance to be measured based on either or both of the level of agglutinate formation and the level of unagglutinated carrier particles; or the steps of:

> (1') applying voltage pulses to a mixed reaction solution comprising an agglutination reagent component, the affinity substance to be measured, and carrier particles that bind to a binding partner having the activity to bind to the affinity substance to be measured, wherein the carrier particles agglutinate via the agglutination reagent and the agglutination is inhibited by the affinity substance to be measred;
>
> (2') counting, after step (1'), agglutinates of carrier particles formed by binding to the agglutination reagent, or unagglutinated carrier particles of which agglutination is inhibited by the binding of the affinity substance to be measured, or both;
>
> (3') determining, after step (2'), the level of the substance to be measured based on either or both of the level of agglutinate formation and the level of unagglutinated carrier particles.

27. The method of claim 26, wherein the step of diluting the reaction solution mixes the reaction solution with a diluent under the condition of voltage pulse application.

28. The method of claim 27, wherein the voltage pulse is an alternating voltage.

29. The method of claim 28, wherein the frequency of the alternating voltage is in the range of 2 KHz to 20 MHz.

30. The method of claim 27, wherein the step of diluting the reaction solution further comprises the step of mixing the reaction solution with a diluent under the condition of voltage pulse application and further diluting the carrier particles after termination of the electric field.

31. The method of claim 27, wherein the step of diluting the reaction solution is a step of diluting the reaction by mixing the reaction solution after addition of a binding enhancer that enhances the binding between the affinity substance to be measured and the binding partner, or the binding between the agglutination reagent and the binding partner, or a step of diluting the reaction solution with a diluent that contains the binding enhancer.

32. The method of claim 26, wherein the step of diluting the reaction solution is a step of diluting the reaction solution by mixing the reaction solution with a diluent after adding to the reaction solution a binding enhancer that enhances the binding between the affinity substance to be measured and the binding partner, or the binding between the agglutination reagent and the binding partner, or a step of diluting the reaction solution with a diluent that contains the binding enhancer.

33. The method of claim 32, wherein the binding between the affinity substance to be measured and the binding partner, or the binding between the agglutination reagent and the binding partner is immunological binding.

34. The method of claim 33, wherein the antigen is a protein antigen and the binding enhancer is a compound that comprises either glutaraldehyde or carbodiimide, or both.

35. The method of claim 32, wherein the step of diluting the reaction solution mixes the reaction solution with a diluent during voltage pulse application.

36. The method of claim 26, wherein the voltage pulse in step (1) or (1') is an alternating voltage pulse.

37. The method of claim 26, wherein voltage pulses are applied several times in step (1) or (1').

38. The method of claim 37, wherein step (1) or (1') comprises dispersing carrier particles and applying subsequent voltage pulses after voltage pulse application.

39. The method of claim 37, wherein the voltage pulses are applies several times in different directions.

40. The method of claim 26, wherein the mean particle size of carrier particles is 1 $\mu$m or greater.

41. The method of claim 40, wherein the mean particle size of carrier particles is in the range of 1 to 20 $\mu$m.

**42.** The method of claim 26, wherein step (2) or (2') counts either or both of agglutinates and unagglutinated carrier particles using three-dimensional information thereof as an indicator.

**43.** The method of claim 42, wherein step (2) or (2') physically measures the three-dimensional information of the agglutinates or carrier particles.

**44.** The method of claim 43, wherein the method that physically measures the three-dimensional information is any one selected from the group consisting of electric resistance method, laser diffraction method, and three dimensional imaging analysis.

**45.** A device for measuring the binding between an affinity substance and carrier particles that bind to a binding partner having the activity to bind to the affinity substance to be measured, using as an indicator agglutination of the carrier particles by the affinity substance or an agglutination reagent, which comprises the elements of:

    a: a space for retaining a reaction solution which comprises a sample comprising the affinity substance to be measured and carrier particles that bind to a binding partner having the activity to bind to the affinity substance to be measured, or the reaction solution further comprising an agglutination reagent;
    b: a means of applying voltage pulses to the reaction solution;
    c: a means of diluting the reaction solution; and
    d: a means of counting either or both of carrier particles and carrier particle agglutinates in the reaction solution.

**46.** The device of claim 45, wherein the means of diluting the reaction solution is a means of mixing the reaction solution with a diluent during voltage pulse application.

**47.** The device of claim 45, wherein the means of diluting the reaction solution comprises a means of adding to the reaction solution a binding enhancer that enhances the binding between the affinity substance to be measured and the binding partner, or the binding between an agglutination reagent and the binding partner.

SAMPLE    REAGENT 2
    REAGENT 1

(A)

(B)

1: DISPENSING STIRRING MEANS    2: TEMPERATURE CONTROL MACHINERY
3: REACTION VESSEL    4: ELECTRODES(PULSE APPLICATION MEANS)
5: DILUTION MEANS    6: MEANS OF MEASURING
    PARTICLE SIZE DISTRIBUTION

# FIG. 1

FIG. 2

FIG. 3

FIG. 4

| 1: DISPENSING STIRRING MEANS | 2: REACTION VESSEL |
|---|---|
| 3: ELECTRODES (PULSE APPLICATION MEANS) | 4: TEMPERATURE CONTROL MACHINERY |
| 5: DILUTION MEANS | 6: MEANS OF MEASURING PARTICLE SIZE DISTRIBUTION |

# FIG. 5

FIG. 6

FIG. 7

FIG. 8

FIG. 9

FIG. 10

(A)

(B)

1: DISPENSING STIRRING MEANS
   (+TEMPERATURE CONTROL MACHINERY)
2: REACTION VESSEL
3: ELECTRODES (PULSE APPLICATION MEANS)
4: TEMPERATURE CONTROL MACHINERY
5: DILUTION MEANS
6: MEANS OF MEASURING
   PARTICLE SIZE DISTRIBUTION

   101: ELECTRODES
   102: HIGH-FREQUENCY POWER SUPPLY
   103: DILUTION VESSEL
   104: DILUENT

FIG. 11

FIG. 12

FIG. 13

FIG. 14

FIG. 15

FIG. 16

FIG. 17

FIG. 18

FIG. 19

FIG. 20

FIG. 21

FIG. 22

## INTERNATIONAL SEARCH REPORT

| | International application No. |
| --- | --- |
| | PCT/JP2005/004234 |

| A. CLASSIFICATION OF SUBJECT MATTER |
| --- |
| Int.Cl⁷ G01N33/543 |

According to International Patent Classification (IPC) or to both national classification and IPC

| B. FIELDS SEARCHED |
| --- |
| Minimum documentation searched (classification system followed by classification symbols) |
| Int.Cl⁷ G01N33/543 |

Documentation searched other than minimum documentation to the extent that such documents are included in the fields searched

| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | JP 10-73596 A  (Masao KARUBE),<br>17 March, 1998 (17.03.98),<br>(Family: none) | 1-47 |
| Y | JP 10-73597 A  (Masao KARUBE),<br>17 March, 1998 (17.03.98),<br>(Family: none) | 1-47 |
| Y | JP 6-174734 A  (Kowa Co., Ltd.),<br>24 June, 1994 (24.06.94),<br>(Family: none) | 1-47 |
| A | JP 2001-337092 A  (Daiichi Pure Chemicals Co., Ltd.),<br>07 December, 2001 (07.12.01),<br>(Family: none) | 13-16,26 |

| [X]  Further documents are listed in the continuation of Box C. | [ ]  See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 22 June, 2005 (22.06.05) | 12 July, 2005 (12.07.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**EP 1 724 583 A1**

**INTERNATIONAL SEARCH REPORT**

| International application No. |
| --- |
| PCT/JP2005/004234 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 11-326327 A  (Shino-Test Corp.),<br>26 November, 1999 (26.11.99),<br>(Family: none) | 31-34 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H783928 A

- JP 7083928 A

### Non-patent literature cited in the description

- **CAMBIASO et al.** *J. Immunol. Methods,* 1977, vol. 18, 33
- **MATSUZAWA et al.** *Kagaku to kogyo,* 1982, vol. 36 (4

- **MATSUZAWA et al.** *Kagaku To Kogyo,* 1982, vol. 36 (4